(19) **Europäisches Patentamt / European Patent Office / Office européen des brevets**

(11) **EP 4 564 369 A1**

(12) **EUROPEAN PATENT APPLICATION**
published in accordance with Art. 153(4) EPC

(43) Date of publication:
04.06.2025 Bulletin 2025/23

(21) Application number: 22952610.8

(22) Date of filing: 11.08.2022

(51) International Patent Classification (IPC):
*G16H 50/20* (2018.01)

(86) International application number:
PCT/CN2022/111753

(87) International publication number:
WO 2024/021166 (01.02.2024 Gazette 2024/05)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 26.07.2022 CN 202210886125

(71) Applicant: **Peking University Third Hospital (The Third Clinical Medical School of Peking University) Beijing 100191 (CN)**

(72) Inventors:
• **XU, Huiyu**
  **Beijing 100191 (CN)**
• **LI, Rong**
  **Beijing 100191 (CN)**
• **QIAO, Jie**
  **Beijing 100191 (CN)**
• **FENG, Guoshuang**
  **Beijing 100191 (CN)**
• **HAN, Yong**
  **Beijing 100191 (CN)**

(74) Representative: **Keller Schneider Patentanwaltsgesellschaft mbH Linprunstraße 10 80335 München (DE)**

(54) **SYSTEM AND METHOD FOR PREDICTING DOSAGE OF ADMINISTERED EXOGENOUS FOLLICLE-STIMULATING HORMONE DRUG IN COS CYCLE**

(57) A system for predicting the dosage of exogenous FSH administered to a subject in a COS cycle, comprising: a data acquisition module used for acquiring the age, basic AMH, basic FSH or ΔINHB, and basic AFC data of the subject; and an exogenous FSH drug dosage calculation module used for: performing first calculation on the acquired data so as to calculate predicted NROs of the subject; performing second calculation on the acquired data so as to calculate the ratio of the predicted NROs of the subject to the dosage of the exogenous FSH drug administered to the subject; and calculating the dosage of the administered exogenous FSH drug on the basis of the predicted NROs obtained by the first calculation and the ratio. In the system, the predicted NROs are first calculated, then predicted ovarian sensitivity, i.e., the ratio of the predicted NROs to the dosage of the exogenous FSH drug, is calculated, and then the dosage of the administered exogenous FSH drug is obtained, so that the dosage of the exogenous FSH drug can be predicted when there is only one unknown variable, i.e., the dosage of the exogenous FSH drug, in the predicted ovarian sensitivity outcome variables.

Figure.1A

EP 4 564 369 A1

**Description**

**TECHNICAL FIELD**

The present application relates to the field of medical technology, and particularly relates to a system and method in which the predicted ovarian sensitivity i.e., the ratio of the predicted number of retrieved oocytes to the dosage of the exogenous follicle-stimulating hormone (FSH) drug, in a controlled ovarian stimulation cycle is calculated, and then the initial dosage and adjusted dosage of the exogenous FSH drug administered to the subject are obtained.

**BACKGROUND**

For women who undergo controlled ovarian stimulation (COS) and in vitro fertilization/intracytoplasmic sperm injection (IVF/ICSI) cycles, the number of retrieved oocytes, (NROs) by COS treatment is considered a powerful surrogate prognostic marker for successful pregnancy. Optimal NROs help improve live-birth-rate (LBR).

Infertility is defined as the failure to conceive after 12 months of regular and unprotected intercourse. The infertility rate of women of childbearing age in China is as high as 12% to 15%. Assisted reproductive technology (ART) is the most common and effective method used to treat infertility. Meanwhile, controlled ovarian stimulation (COS), in vitro fertilization (IVF) and embryo transfer (ET) are the most common and effective types of ART. Personalized COS is a milestone in the history of ART. Choosing the appropriate dosage of exogenous follicle-stimulating hormone (FSH) is critical for COS. There are two important time points for personalized COS, one is when the initial dosage is selected at the beginning of each new treatment cycle, and the other is when dosage adjustments are made within a given COS cycle.

The selection of the initial dosage for ovulation induction treatment is very important. In the past, clinical doctors often combined the size and number of follicles under ultrasound during treatment with growth changes of LH (luteinizing hormone), estradiol (E2), and progesterone (P) to estimate the predicted number of retrieved oocytes and adjust the dosage of ovulation induction drugs based on personal experience. However, up to now, the adjustment of the dosage of exogenous FSH drugs during ovulation induction mainly relies on subjective experience and there is no unified standard internationally. The present research team has previously developed a system and method that uses basic ovarian reserve indicators (indicators before ovulation induction treatment) to predict ovarian response to ovulation induction treatment. The outcome variable of this system is the probability of poor ovarian response, and people with similar probability of the poor ovarian response are grouped together, and dosage recommendations are given. Although this dosage recommendation is somewhat advanced, there is no dosage in the outcome variable. Its essence is a combination of predicting the probability of poor ovarian response and clinical experience. Although it is a common practice internationally, but it obviously involves human experience and judgment. It is not intelligent enough and deserves further improvement.

In addition, some researchers have used models to predict the number of retrieved oocytes (NROs), and then combined the predicted NROs with clinical hypotheses to recommend a certain starting dosage of exogenous FSH in patients with similar ovarian responses. The main outcome variable in these models is NROs. La Marca et al. proposed a new idea to predict the starting dosage of exogenous FSH drugs by using the ratio of actual NROs to the actual initial dosage as the outcome variable. For the first time, they included dosage in the outcome variable, that is, dosage is incorporated into the model rather than being judged based on experience. They used three basic indicators of age, FSH and anti-Müllerian hormone (AMH) to predict ovarian sensitivity, i.e., actual NROs divided by the actual initial dosage of exogenous FSH, and the model's $R^2$ was 0.3. However, the outcome variable is the ratio of actual NROs to the initial dosage of exogenous FSH, both of which are unknown prior to ovarian stimulation, must be assumed before final calculation of dosage. They assumed that all individuals had NROs of 9 and then calculated the initial dosage of exogenous FSH. Although this study has great innovative value for predicting the starting dosage of exogenous FSH compared with previous models, fixing NROs at 9 lacks individualized guidance and is not consistent with the actual situation of most patients.

**SUMMARY**

Choosing the appropriate dosage of an exogenous follicle-stimulating hormone (FSH) drug is the key to controlled ovarian stimulation (COS). Standard fixed doses of exogenous FSH are not suitable for all women because of differences in their ovarian reserve and ovarian response. The most appropriate starting dosage and adjusted dosages of the exogenous FSH drug is determined based on individualized ovarian reserve and ovarian drug response to exogenous FSH, which has been a goal for many physicians. To date, no simple and applicable online tool has been developed. Accordingly, the present application provides a system and method for predicting the initial dosage and adjusted dosage of the exogenous FSH drug administered to a subject during a controlled ovarian stimulation cycle based on individualized ovarian reserve and response to the exogenous FSH drug. The prediction of initial dosage and adjusted dosage can be realized based on basic indicators. The $R^2$ of the prediction model in the present application is greater than 0.9, which is much higher than that of the existing technology model.

To sum up, the present application involves the following contents:

1. A system for predicting a dosage of an exogenous follicle-stimulating hormone (FSH) drug to be administered to a subject in a controlled ovarian stimulation cycle, comprising:

a data collection module, which is used for acquiring data of age, basal anti-Müllerian hormone (AMH) level, a basal follicle-stimulating hormone (FSH) level or a dynamic change in inhibin B level ($\Delta$INHB), and basal antral follicle count (AFC) of the subject; and

a calculation module for obtaining the dosage of an exogenous follicle-stimulating hormone (FSH) drug, which is used for performing a first calculation on the data acquired by the data collection module, so as to calculate predicted number of retrieved oocytes (predicted NROs) of the subject in an ovulation induction cycle; performing a second calculation on the data acquired by the data collection module, so as to calculate a ratio of the predicted number of retrieved oocytes (predicted NROs) of the subject in the ovulation induction cycle to the dosage of the exogenous FSH drug, i.e., predicted ovarian sensitivity; and calculating the dosage of the exogenous FSH drug to be administered to the subject based on the predicted number of retrieved oocytes (predicted NROs) obtained by the first calculation and the ratio obtained by the second calculation.

2. The system according to item 1, wherein
the subject is a subject who will receive standard ovulation induction treatment, and the number of retrieved oocytes (NROs) of the subject is the number of mature oocytes with a diameter of 10 mm or more, preferably 15 mm or more; wherein the mature oocytes are obtained after the subject firstly receives ovulation induction treatment and the diameter of one or two dominant follicles reaches 18 mm or more during the ovarian stimulation and then the subject undergoes hCG injection for inducing follicle maturation.

3. The system according to item 1 or 2, wherein
in the data collection module, the basal anti-Müllerian hormone (AMH) level acquired refers to a concentration of anti-Müllerian hormone in venous blood of the subject at any time point during a menstrual period before ovulation induction treatment.

4. The system according to any one of items 1 to 3, wherein
in the data collection module, the basal follicle-stimulating hormone (FSH) level acquired refers to a concentration of follicle-stimulating hormone in venous blood of the female subject on day 2 to day 4 of menstruation before ovulation induction treatment.

5. The system according to any one of items 1 to 4, wherein
in the data collection module, the basal antral follicle count (AFC) acquired refers to the number of all visible follicles with a diameter of 2 to 10 mm in two ovaries of the female subject on day 2 of menstruation counted by transvaginal B-mode ultrasound.

6. The system according to any one of items 1 to 5, wherein
in the data collection module, the dynamic change in inhibin B level ($\Delta$INHB) acquired refers to dynamic change in inhibin B level ($\Delta$INHB) in an early stage of ovulation induction treatment, preferably a difference between a serum inhibin B concentration on day 6 of menstruation and an inhibin B concentration in venous blood on day 2 of menstruation of the female subject in a cycle of ovulation induction treatment by GnRH antagonist regimen.

7. The system according to any one of items 1 to 6, wherein

the data collection module is used for acquiring data of the age, the basal anti-Müllerian hormone (AMH) level, the basal follicle-stimulating hormone (FSH) level, and the basal antral follicle count (AFC) of the subject; and;
the calculation module for obtaining the dosage of the exogenous follicle-stimulating hormone (FSH) drug is used for performing a first calculation on the data acquired by the data collection module, so as to calculate predicted the number of retrieved oocytes (predicted NROs) of the subject in the ovulation induction cycle; performing a second calculation on the data acquired by the data collection module, so as to calculate a ratio of the predicted number of retrieved oocytes (predicted NROs) of the subject in the ovulation induction cycle to an initial dosage of the exogenous FSH drug, i.e., predicted ovarian sensitivity; and calculating the initial dosage of the exogenous FSH drug to be administered to the subject based on the predicted number of retrieved oocytes (predicted NROs) obtained by the first calculation and the ratio obtained by the second calculation.

8. The system according to item 7, wherein
in the calculation module for obtaining the dosage of the exogenous FSH drug, formula I for calculating the predicted number of retrieved oocytes (predicted NROs) of the subject is pre-stored, wherein the formula I is obtained by fitting based on data of the age, the basal anti-Müllerian hormone (AMH) level, the basal follicle-stimulating hormone (FSH), the basal antral follicle count (AFC) and the actual number of retrieved oocytes of a patient who has received ovulation induction treatment by standard GnRH antagonist regimen in an existing database.

9. The system according to item 8, wherein

in the calculation module for obtaining the dosage of the exogenous FSH drug, the formula I is a calculation formula I obtained by using negative binomial distribution of the outcome variable to fit the data of the age, the basal anti-Müllerian hormone (AMH) level, the basal follicle-stimulating hormone (FSH), the basal antral follicle count (AFC) and the actual number of retrieved oocytes (outcome variable) of the patient who has received ovulation induction treatment by standard GnRH antagonist regimen in the existing database, using negative binomial distribution of the outcome variable; and
the formula I can be used to calculate the predicted number of retrieved oocytes (predicted NROs) of the subject by using the data of the age, the basal anti-Müllerian hormone (AMH) level, the basal follicle-stimulating hormone (FSH) level, and the basal antral follicle count (AFC) of the subject acquired by the data collection module.

10. The system according to item 9, wherein the formula I is:

$$\text{the predicted NROs} = \text{EXP}(a+b*\text{age}*+c*\text{basal FSH}+d*\text{LN[basal AMH]}+f*\text{LN[basal AFC]});$$

wherein a is any value selected from 1.5576128 to 2.6037078, preferably 2.0806603;
b is any value selected from -0.019097 to 0.0044064, preferably -0.007345;
c is any value selected from -0.045234 to -0.004054, preferably -0.024644;
d is any value selected from 0.348168 to 0.4948875, preferably 0.4215277; and
f is any value selected from 0.0415663 to 0.2566199, preferably 0.1490931.

11. The system according to item 10, wherein

in the calculation module for obtaining the dosage of the exogenous FSH drug, formula II for calculating the predicted ovarian sensitivity of the subject, i.e., the ratio of the predicted number of retrieved oocytes (predicted NROs) to the initial dosage of the exogenous FSH drug is pre-stored, wherein the formula II is obtained by fitting based on the data of the age, the basal anti-Müllerian hormone (AMH) level, the basal follicle-stimulating hormone (FSH), and the basal antral follicle count (AFC) of the patient who has received ovulation induction treatment by standard GnRH antagonist regimen in the existing database, and a ratio of the predicted number of retrieved oocytes (predicted NROs) calculated by formula I to an average daily dosage of the exogenous FSH drug used by the patient;
wherein the average daily dosage of the exogenous FSH drug used by the patient refers to a ratio of a total dosage of the exogenous FSH drug used by the patient to the number of days of using the exogenous FSH drug during the past ovulation induction treatment by standard GnRH antagonist regimen.

12. The system according to item 11, wherein
in the calculation module for obtaining the dosage of the exogenous FSH drug, the formula II can be used to calculate the predicted ovarian sensitivity of the subject, i.e., the ratio of the predicted numbers of retrieved oocytes (predicted NROs) to the initial dosage of the exogenous FSH drug to be administrated to the subject, by using the data of the age, the basal anti-Müllerian hormone (AMH) level, the basal follicle-stimulating hormone (FSH) level, and the basal antral follicle count (AFC) of the subject acquired by the data collection module.

13. The system according to item 11 or 12, wherein the formula II is:

the predicted ovarian sensitivity of the subject, i.e., the predicted NROs calculated by formula I/ the initial dosage of the exogenous FSH drug = EXP(g+h*age+i*basal FSH+j*LN[basal AMH]+k*basal AFC)
wherein g is any value selected from -3.167587 to -2.751518, preferably -2.959552;
h is any value selected from -0.025951 to -0.016355, preferably -0.021153;
i is any value selected from -0.048143 to -0.025727, preferably -0.036935;
j is any value selected from 0.5174476 to 0.6075545, preferably 0.5625011; and
k is any value selected from 0.0241595 to 0.0365579, preferably 0.0303587.

14. The system according to item 13, wherein based on the predicted numbers of retrieved oocytes (predicted NROs) obtained by the first calculation and the ratio obtained by the second calculation, the initial dosage of the exogenous FSH drug to be administered to the subject is calculated by using formula III, which is:

The initial dosage of the exogenous FSH drug = Round (the predicted NROs calculated by formula I/the predicted ovarian sensitivity calculated by formula II, 0).

15. The system according to any one of items 1 to 6, wherein

the data collection module is used for acquiring data of the age, the basal anti-Miillerian hormone (AMH) level, the basal antral follicle count (AFC) and the dynamic change in inhibin B level of the subject; and

the calculation module for obtaining the dosage of the exogenous follicle-stimulating hormone (FSH) drug is used for performing a first calculation on the data acquired by the data collection module, so as to calculate the predicted numbers of retrieved oocytes (predicted NROs) of the subject in the ovulation induction cycle; performing a second calculation on the data acquired by the data collection module, so as to calculate a ratio of the predicted number of retrieved oocytes (predicted NROs) of the subject in the ovulation induction cycle to an adjusted dosage of the exogenous FSH drug, i.e., predicted ovarian sensitivity; and calculating the adjusted dosage of the exogenous FSH drug to be administered to the subject based on the predicted NROs obtained by the first calculation and the ratio obtained by the second calculation.

16. The system according to item 15, wherein

in the calculation module for obtaining the dosage of the exogenous FSH drug, formula IV for calculating the predicted number of retrieved oocytes (predicted NROs) of the subject is pre-stored, wherein the formula IV is obtained by fitting based on the data of the age, the basal anti-Miillerian hormone (AMH) level, the basal antral follicle count (AFC), the dynamic change in inhibin B level ($\Delta$INHB), and the actual number of retrieved oocytes of the patient who has received ovulation induction treatment by standard GnRH antagonist regimen in the existing database.

17. The system according to item 16, wherein

in the calculation module for obtaining the dosage of the exogenous FSH drug, the formula IV is a calculation formula IV which is obtained by fitting the data of the age, the basal anti-Müllerian hormone (AMH) level, the basal antral follicle count (AFC), the dynamic change in inhibin B level ($\Delta$INHB), and the actual number of retrieved oocytes (outcome variable) of the patient who has received ovulation induction treatment by standard GnRH antagonist regimen in the existing database, using negative binomial distribution of the outcome variable; and

the formula IV can be used to calculate the predicted number of retrieved oocytes (predicted NROs) of the subject by using the data of the age, the basal anti-Miillerian hormone (AMH) level, the basal antral follicle count (AFC) and the dynamic change in inhibin B level ($\Delta$INHB) of the subject acquired by the data collection module.

18. The system according to item 17, wherein the formula IV is:

the predicted NROs=EXP(w+m*age*+n*LN[basal AMH]+o*LN[$\Delta$INHB]+p*LN[basal AFC]);

wherein w is any value selected from -0.447201 to 0.9161863, preferably 0.2344927;

m is any value selected from -0.017165 to 0.0039328, preferably -0.006616;

n is any value selected from 0.1318094 to 0.3113979, preferably 0.2216036;

o is any value selected from 0.1901643 to 0.3850919, preferably 0.2876281; and

p is any value selected from 0.0541966 to 0.2338079, preferably 0.1440023.

19. The system according to item 18, wherein,

in the calculation module for obtaining the dosage of the exogenous FSH drug, formula V for calculating the predicted ovarian sensitivity of the subject, i.e., the ratio of the predicted number of retrieved oocytes (predicted NROs) to the adjusted dosage of the exogenous FSH drug, is pre-stored; wherein the formula V is obtained by fitting based on the data of the age, the basal anti-Miillerian hormone (AMH) level, the basal antral follicle count (AFC), and the dynamic change in inhibin B level ($\Delta$INHB) of the patient who has received ovulation induction treatment by standard GnRH antagonist regimen in the existing database, and a ratio of the predicted number of retrieved oocytes (predicted NROs) calculated by formula IV to an average daily dosage of the exogenous FSH drug used by the patient,

wherein the average daily dosage of the exogenous FSH drug used by the patient refers to a ratio of a total dosage of the exogenous FSH drug used by the patient to the number of days of using the exogenous FSH drug during the past ovulation induction treatment by standard GnRH antagonist regimen.

20. The system according to item 19, wherein,

in the calculation module for obtaining the dosage of the exogenous FSH drug, the formula V can be used to calculate the predicted ovarian sensitivity of the subject, i.e., the ratio of the predicted number of retrieved oocytes (predicted NROs) to the adjusted dosage of the exogenous FSH drug to be administrated to the subject, by using the data of the age, the basal anti-Müllerian hormone (AMH) level, the basal antral follicle count (AFC) and the dynamic change in inhibin B level (ΔINHB) of the subject acquired by the data collection module.

21. The system according to item 19 or 20, wherein the formula V is:

the predicted ovarian sensitivity of the subject, i.e., the predicted NROs calculated by formula IV/ the adjusted dosage of the exogenous FSH drug = EXP(q+r*age+s*LN[basal AMH]+t*basal AFC+u*LN[ΔINHB])

wherein q is any value selected from -5.63461 to -5.108612, preferably -5.371611;

r is any value selected from -0.0264 to -0.015183, preferably -0.020792;

s is any value selected from 0.2696292 to 0.3684551, preferably 0.3190421;

t is any value selected from 0.0273504 to 0.0399372, preferably 0.0336438; and

u is any value selected from 0.3327566 to 0.3940448, preferably 0.3634007.

22. The system according to item 21, wherein based on the predicted number of retrieved oocytes (predicted NROs) obtained by the first calculation and the ratio obtained by the second calculation, the adjusted dosage of the exogenous FSH drug to be administered to the subject is calculated using Formula VI which is:

the adjusted dosage of the exogenous FSH drug = Round (the predicted NROs calculated by formula VI/the predicted ovarian sensitivity calculated by formula V, 0).

23. A method for predicting a dosage of an exogenous FSH drug to be administered to a subject in a controlled ovarian stimulation cycle, comprising:

a data collection step, in which data of age, basal anti-Miillerian hormone (AMH) level, a basal follicle-stimulating hormone (FSH) level or adynamic change in inhibin B level (ΔINHB), and basal antral follicle count (AFC) of the subject is acquired; and

a calculation step for obtaining the dosage of an exogenous FSH drug, in which a first calculation is performed on the data acquired in the data collection step, so as to calculate predicted number of retrieved oocytes (predicted NROs) of the subject in an ovulation induction cycle; a second calculation is performed on the data acquired in the data collection step, so as to calculate a ratio of the predicted number of retrieved oocytes (predicted NROs) of the subject in the ovulation induction cycle to the dosage of the exogenous FSH drug, i.e., predicted ovarian sensitivity; and the dosage of the exogenous FSH drug to be administered to the subject is calculated based on the predicted number of retrieved oocytes (predicted NROs) obtained by the first calculation and the ratio obtained by the second calculation.

24. The method according to item 23, wherein

the subject is a subject who will receive standard ovulation induction treatment, and the number of retrieved oocytes (NROs) of the subject is the number of mature oocytes with a diameter of 10 mm or more, preferably 15 mm or more; wherein the mature oocytes are obtained after the subject firstly receives ovulation induction treatment and the diameter of one or two dominant follicles reaches 18 mm or more during the ovarian stimulation and then the subject undergoes hCG injection for inducing follicle maturation.

25. The method according to item 23 or 24, wherein

in the data collection step, the basal anti-Müllerian hormone (AMH) level acquired refers to a concentration of anti-Müllerian hormone in venous blood of the subject at any time point during a menstrual period before ovulation induction treatment.

26. The method according to any one of items 23 to 25, wherein

in the data collection step, the basal follicle-stimulating hormone (FSH) level acquired refers to a concentration of follicle-stimulating hormone in venous blood of the female subject on day 2 to day 4 of menstruation before ovulation induction treatment.

27. The method according to any one of items 23 to 26, wherein

in the data collection step, the basal antral follicle count (AFC) acquired refers to the number of all visible follicles with a diameter of 2 to 10 mm in two ovaries of the female subject on day 2 of menstruation counted by transvaginal B-mode ultrasound.

28. The method according to any one of items 23 to 27, wherein

in the data collection step, the dynamic change in inhibin B level (ΔINHB) acquired refers to a dynamic change in inhibin B level (ΔINHB) in an early stage of ovulation induction treatment, preferably a difference between a serum inhibin B concentration on day 6 of menstruation and an inhibin B concentration in venous blood on day 2 of menstruation of the female subject in a cycle of ovulation induction treatment by GnRH antagonist regimen.

29. The method according to any one of items 23 to 28, wherein

in the data collection step, data of the age, the basal anti-Müllerian hormone (AMH) level, the dynamic change in inhibin B level (∆INHB), and the basal antral follicle count ( AFC) of the subject is acquired; and
in the calculation step for obtaining the dosage of an exogenous FSH drug, a first calculation is performed on the data acquired by the data collection step, so as to calculate the predicted number of retrieved oocytes (predicted NROs) of the subject in the ovulation induction cycle; a second calculation is performed on the data acquired by the data collection step, so as to calculate a ratio of the predicted number of retrieved oocytes (predicted NROs) of the subject in the ovulation induction cycle to an adjusted dosage of the exogenous FSH drug, i.e., predicted ovarian sensitivity; and the adjusted dosage of the exogenous FSH drug to be administered to the subject is calculated based on the predicted number of retrieved oocytes (predicted NROs) obtained by the first calculation and the ratio obtained by the second calculation.

30. The method according to item 29, wherein
in the calculation step for obtaining the dosage of the exogenous FSH drug, formula I for calculating the predicted number of retrieved oocytes (predicted NROs) of the subject is pre-stored, wherein the formula I is obtained by fitting based on data of the age, the basal anti-Müllerian hormone (AMH) level, the dynamic change in inhibin B level (∆INHB), the basal antral follicle count (AFC) and the actual number of retrieved oocytes of a patient who has received ovulation induction treatment by standard GnRH antagonist regimen in an existing database.

31. The method according to item 30, wherein,

in the calculation step for obtaining the dosage of the exogenous FSH drug, the formula I is a calculation formula I obtained by using negative binomial distribution of the outcome variable to fit the data of the age, the basal anti-Müllerian hormone (AMH) level, the basal follicle-stimulating hormone (FSH), the basal antral follicle count (AFC) and the actual number of retrieved oocytes (outcome variable) of the patient who has received ovulation induction treatment by standard GnRH antagonist regimen in the existing database, using negative binomial distribution of the outcome variable; and
the formula I can be used to calculate the predicted number of retrieved oocytes (predicted NROs) of the subject by using the data of the age, the basal anti-Müllerian hormone (AMH) level, the basal follicle-stimulating hormone (FSH) level, and the basal antral follicle count (AFC) of the subject acquired by the data collection step.

32. The method according to item 31, wherein the formula I is:

$$\text{the predicted NROs} = EXP(a + b*age* + c*basal\ FSH + d*LN[basal\ AMH] + f*LN[basal\ AFC]);$$

wherein a is any value selected from 1.5576128 to 2.6037078, preferably 2.0806603;
b is any value selected from -0.019097 to 0.0044064, preferably -0.007345;
c is any value selected from -0.045234 to -0.004054, preferably -0.024644;
d is any value selected from 0.348168 to 0.4948875, preferably 0.4215277; and
f is any value selected from 0.0415663 to 0.2566199, preferably 0.1490931.

33. The method according to item 32, wherein

in the calculation step for obtaining the dosage of the exogenous FSH drug, formula II for calculating the predicted ovarian sensitivity of the subject, i.e. the ratio of the predicted number of retrieved oocytes (predicted NROs) to the initial dosage of the exogenous FSH drug is pre-stored, wherein the formula II is obtained by fitting based on the data of the age, the basal anti-Müllerian hormone (AMH) level, the basal follicle-stimulating hormone (FSH) , and the basal antral follicle count (AFC) of the patient who has received ovulation induction treatment by standard GnRH antagonist regimen in the existing database, and a ratio of the predicted number of retrieved oocytes (predicted NROs) calculated by formula I to an average daily dosage of the exogenous FSH drug used by the patient;
wherein the average daily dosage of the exogenous FSH drug used by the patient refers to a ratio of a total dosage of the exogenous FSH drug used by the patient to the number of days of using the exogenous FSH drug during the past ovulation induction treatment by standard GnRH antagonist regimen.

34. The method according to item 33, wherein

in the calculation step for obtaining the dosage of the exogenous FSH drug, the formula II can be used to calculate the predicted ovarian sensitivity of the subject, i.e., the ratio of the predicted numbers of retrieved oocytes (predicted NROs) to the initial dosage of the exogenous FSH drug to be administrated to the subject, by using the data of the age, the basal anti-Müllerian hormone (AMH) level, the basal follicle-stimulating hormone (FSH) level, and the basal antral follicle count (AFC) of the subject acquired by the data collection step.

35. The method according to item 33 or 34, wherein the formula II is:

the predicted ovarian sensitivity of the subject, i.e., the predicted NROs calculated by formula I/ the initial dosage of the exogenous FSH drug= EXP(g+h*age+i*basal FSH+j*LN[basal AMH]+k*basal AFC)
wherein g is any value selected from -3.167587 to -2.751518, preferably -2.959552;
h is any value selected from -0.025951 to -0.016355, preferably -0.021153;
i is any value selected from -0.048143 to -0.025727, preferably -0.036935;
j is any value selected from 0.5174476 to 0.6075545, preferably 0.5625011; and
k is any value selected from 0.0241595 to 0.0365579, preferably 0.0303587.

36. The method according to item 35, wherein based on the predicted numbers of retrieved oocytes (predicted NROs) obtained by the first calculation and the ratio obtained by the second calculation, the initial dosage of the exogenous FSH drug to be administered to the subject is calculated by using formula III, which is:
the initial dosage of the exogenous FSH drug = Round (the predicted NROs calculated by formula I/the predicted ovarian sensitivity calculated by formula II, 0).

37. The method according to any one of items 23 to 28, wherein

in the data collection step, data of the age, the basal anti-Müllerian hormone (AMH) level, the basal antral follicle count (AFC) and the dynamic change in inhibin B level (ΔINHB) of the subject is acquired; and
in the calculation step for obtaining the dosage of the exogenous FSH drug, a first calculation is performed on the data acquired by the data collection module, so as to calculate the predicted number of retrieved oocytes (predicted NROs) of the subject in the ovulation induction cycle; a second calculation is performed on the data acquired by the data collection step, so as to calculate a ratio of the predicted number of retrieved oocytes (predicted NROs) of the subject in the ovulation induction cycle to an adjusted dosage of the exogenous FSH drug, i.e., predicted ovarian sensitivity; and the adjusted dosage of the exogenous FSH drug to be administered to the subject is calculated based on the predicted number of retrieved oocytes (predicted NROs) obtained by the first calculation and the ratio obtained by the second calculation.

38. The method according to item 37, wherein
in the calculation step for obtaining the dosage of the exogenous FSH drug, formula IV for calculating the predicted number of retrieved oocytes (predicted NROs) of the subject is pre-stored, wherein the formula IV is obtained by fitting based on the data of the age, the basal anti-Müllerian hormone (AMH) level, the basal antral follicle count (AFC) and the dynamic change in inhibin B level (ΔINHB), and the actual number of retrieved oocytes of a patient who has received ovulation induction treatment by standard GnRH antagonist regimen in the existing database.

39. The method according to item 38, wherein

in the calculation step for obtaining the dosage of the exogenous FSH drug, the formula IV is a calculation formula IV which is obtained by fitting the data of the age, the basal anti-Müllerian hormone (AMH) level, the basal antral follicle count (AFC) and the dynamic change in inhibin B level (ΔINHB), and the actual number of retrieved oocytes (outcome variable) of the patient who has received ovulation induction treatment by standard GnRH antagonist regimen in the existing database, using negative binomial distribution of the outcome variable;
the formula IV can be used to calculate the predicted number of retrieved oocytes (predicted NROs) of the subject by using the data of the age, the basal anti-Müllerian hormone (AMH) level, the basal antral follicle count (AFC) and the dynamic change in inhibin B level (ΔINHB) of the subject acquired by the data collection step.

40. The method according to item 39, wherein the formula IV is:

the predicted NROs=EXP(w+m*age*+n*LN[basal AMH]+o*LN[ΔINHB]+p*LN[basal AFC]);

wherein w is any value selected from -0.447201 to 0.9161863, preferably 0.2344927;
m is any value selected from -0.017165 to 0.0039328, preferably -0.006616;

n is any value selected from 0.1318094 to 0.3113979, preferably 0.2216036;

o is any value selected from 0.1901643 to 0.3850919, preferably 0.2876281; and

p is any value selected from 0.0541966 to 0.2338079, preferably 0.1440023.

41. The method according to item 40, wherein

in the calculation step for obtaining the dosage of the exogenous FSH drug, formula V for calculating the predicted ovarian sensitivity of the subject, i.e. the ratio of the predicted number of retrieved oocytes (predicted NROs) to the adjusted dosage of the exogenous FSH drug, is pre-stored; wherein the formula V is obtained by fitting based on the data of the age, the basal anti-Müllerian hormone (AMH) level, the basal antral follicle count (AFC), and the dynamic change in inhibin B level ($\Delta$INHB) of the patient who has received ovulation induction treatment by standard GnRH antagonist regimen in the existing database, and a ratio of predicted number of retrieved oocytes (predicted NROs) calculated by formula IV to an average daily dosage of the exogenous FSH drug used by the patient,

wherein the average daily dosage of the exogenous FSH drug used by the patient refers to a ratio of a total dosage of the exogenous FSH drug used by the patient to the number of days of using the exogenous FSH drug during the past ovulation induction treatment by standard GnRH antagonist regimen.

42. The method according to item 41, wherein

in the calculation step for obtaining the dosage of the exogenous FSH drug, the formula V can be used to calculate the predicted ovarian sensitivity of the subject, i.e., the ratio of the predicted number of retrieved oocytes (predicted NROs) to the adjusted dosage of the exogenous FSH drug to be administrated to the subject, by using the data of the age, the basal anti-Müllerian hormone (AMH) level, the basal antral follicle count (AFC) and the dynamic change in inhibin B level ($\Delta$INHB) of the subject acquired by the data collection step.

43. The method according to item 41 or 42, wherein the formula V is:

the predicted ovarian sensitivity of the subject, i.e., the predicted NROs calculated by formula IV/ the adjusted dosage of the exogenous FSH drug = EXP(q+r*age+s*LN[basal AMH]+t*basal AFC+u*LN[$\Delta$INHB])

wherein q is any value selected from -5.63461 to -5.108612, preferably -5.371611;

r is any value selected from -0.0264 to -0.015183, preferably -0.020792;

s is any value selected from 0.2696292 to 0.3684551, preferably 0.3190421;

t is any value selected from 0.0273504 to 0.0399372, preferably 0.0336438; and

u is any value selected from 0.3327566 to 0.3940448, preferably 0.3634007.

44. The method according to item 43, wherein based on the predicted number of retrieved oocytes (predicted NROs) obtained by the first calculation and the ratio obtained by the second calculation, the adjusted dosage of the exogenous FSH drug to be administered to the subject is calculated using Formula VI which is:

the adjusted dosage of the exogenous FSH drug = Round (the predicted NROs calculated by formula VI/the predicted ovarian sensitivity calculated by formula V, 0).

Effect of the application

In the system or method of the present application, a model for predicting the number of mature oocytes obtained by a subject during an ovulation induction cycle is firstly established based on several basic indicators, and then a model for predicting ovarian sensitivity is established, so that the prediction of dosage can be achieved when there is only one unknown variable, i.e., the dosage of the exogenous FSH drug, in the outcome variable of ovarian sensitivity. The $R^2$ of model is greater than 0.9, which means that the algorithm of the present application can explain more than 90% of the ovarian sensitivity. According to our knowledge, this is the best model for predicting ovarian sensitivity in the world. The use of this algorithm may change the clinical routine of COS in the near future, which will help to improve pregnancy outcomes, reduce the incidence of OHSS and lower the cost during COS, and is expected to greatly improve the therapeutic efficacy of ART, especially improving the treatment homogeneity of ART doctors, while accelerating the learning curve of doctors. The method or system of the present application can achieve prediction of initial dosage and adjusted dosage based on basal indicators. The method or system involved in the present application can be used to guide the initial dosage and adjusted dosage of the exogenous FSH used by an individual during ovulation induction.

BRIEF DESCRIPTION OF THE DRAWINGS

Various other advantages and benefits of the present application will be obvious to those of ordinary skill in the art upon reading the following detailed description of the preferred embodiments. The drawings are only for the purpose of illustrating preferred embodiments and not considered to be limitations of the present application. Obviously, the drawings described below are only some embodiments of the present application. For those of ordinary skill in the art, other drawings can be obtained based on these drawings without creative efforts. In all the drawings, the same reference numerals are used to designate the same components.

FIG. 1A shows the skewed distribution diagram of the outcome variables fitted in model II.
FIG. 1B shows the normal distribution diagram of the outcome variables fitted in model II.
FIG. 1C shows a graph of the nonlinear relationship between AMH and outcome variable.
FIGS. 2A and 2B show process diagrams of the screening of independent variable in model II.
FIG. 2C shows the contribution evaluation results of the four predictors in model II.
FIG. 2D shows a scatter plot of the prediction effect of model II in the training set.
FIG. 2E shows a scatter plot of the prediction effect of model II in the validation set.
FIG. 2F shows the residual plot of the prediction effect of model II in the training set.
FIG. 2G shows a residual plot of the prediction effect of model II in the validation set.
FIGS. 3A and 3B show process diagrams of the screening of independent variables in model IV.
FIG. 3C shows the contribution evaluation results of the four predictors in model IV.
FIG. 3D shows a scatter plot of the prediction effect of model IV in the training set.
FIG. 3E shows a scatter plot of the prediction effect of model IV in the validation set.
FIG. 3F shows a residual plot of the prediction effect of model IV in the training set.
FIG. 3G shows a residual plot of the prediction effect of model IV in the validation set.

## DETAILED DESCRIPTION

Specific embodiments of the present application will be described in more detail below with reference to the accompanying drawings. Although specific embodiments of the present application are shown in the drawings, it should be understood that the present application may be implemented in various forms and should not be limited to the embodiments set forth herein. Rather, these embodiments are provided to provide a thorough understanding of the present application, and to fully convey the scope of the present application to those skilled in the art.

It should be noted that the "comprise" or "include" mentioned throughout the description and claims is an open-ended term, and therefore should be interpreted as " including but not limited to" The following explanations in the description are preferred implementation modes for implementing the present application. However, the explanations are for the purpose of general principles of the description and are not intended to limit the scope of the present application. The scope of protection of the present application shall be determined by the appended claims.

Variable types: in statistics, variable types can be divided into two types: quantitative variables and qualitative variables (also called categorical variables).

Quantitative variables are variables used to describe the quantity and number of things, and can be divided into continuous and discrete types. A continuous variable refers to a variable that can take any value within a certain range, and its value is continuous and can have decimal points. For example, blood pressure, blood sugar, and anthropometric height, weight, chest circumference, etc. are continuous variables, and their values can only be obtained by measuring or metering methods. A discrete variable refers to a variable whose values can only be natural numbers or integer units. For example, the pain score, the number of transferred lesions, the number of retrieved oocytes, etc. can only be positive numbers and cannot take decimal points. The values of such variable are generally obtained by counting methods.

The types of variables are not invariable. According to the needs of research purposes, various types of variables can be converted each other. For example, the amount of hemoglobin (g/L) is originally a numerical variable. If it is divided into two categories according to normal and low hemoglobin, it can be analyzed by binomial categorical data; if it is divided into five ranks according to severe anemia, moderate anemia, mild anemia, normal, and increased hemoglobin, it can be analyzed by ranked data. Sometimes categorical data can also be quantified, for example, the patient's nausea response can be expressed as 0, 1, 2, or 3, and it can be analyzed by numerical variable data (quantitative data).

Outcome variables, also called result variables, or simply outcomes, refer to the predicted result events that will occur during follow-up observation, that is, the events that the researcher hopes to track and observe.

The cause variable refers to the factor or condition that causes the result variable to change due to the active manipulation of the researcher. Therefore, the cause variable is regarded as the cause of the result variable.

The Poisson distribution is a discrete probability distribution commonly seen in statistics and probability. The Poisson distribution is suitable for describing the number of random events occurring per unit time (or space), such as the number of disease cases that appear in a certain space and time, the number of recurrences of a certain disease, the number of metastasis sites of a certain lesion, the number of vomiting times of a certain patient, etc.

The negative binomial distribution is a discrete probability distribution in statistics. The distribution that satisfies the following conditions is called the negative binomial distribution: the experiment contains a series of independent experiments, each experiment has two results, success and failure, the probability of success is constant, and the experiment continues until r times of success, wherein r is a positive integer. The negative binomial distribution is similar to the Poisson distribution and can also be used to describe the relative frequency of a rare event in a certain unit of time and space. The difference from the Poisson distribution is that the Poisson distribution can only be used to describe independent events, while the negative binomial distribution is often used to describe aggregated events, such as the distribution of snails in the soil, the distribution of an infectious disease, etc. Usually, if the mean value of the counting data is found to be greater than the variance, then the Poisson distribution often does not have a good fitting effect, and the negative binomial distribution can be considered.

The normal distribution is a probability distribution in statistics. The normal distribution is a distribution of continuous random variables with two parameters $\mu$ and $\sigma2$. The first parameter $\mu$ is the mean value of a random variable that follows the normal distribution, and the second parameter $\sigma2$ is the variance of this random variable, so the normal distribution is recorded as $N(\mu, \sigma2)$. The probability rule of a random variable that follows a normal distribution is that the probability of taking a value near $\mu$ is high, and the probability of taking a value that is far away from $\mu$ is smaller; the smaller the value, the more concentrated the distribution is near $\mu$, and the larger the value, the more dispersed the distribution. The characteristics of the density function of the normal distribution are that it is symmetric about $\mu$, reaches a maximum value at $\mu$, takes a value of 0 at positive (negative) infinity, and has an inflection point at $\mu\pm$. Its shape is high in the middle and low on both sides, and the image is a bell-shaped curve above the x-axis. When $\mu=0$, $\sigma2=1$, it is called standard normal distribution, recorded as $N(0,1)$.

In the present application, anti-Mullerian hormone (AMH) refers to a hormone secreted by the granulosa cells of small ovarian follicles. Female babies begin to produce AMH during fetal period. The more small follicles in the ovary, the higher the concentration of AMH; conversely, when follicles are gradually consumed with age and various factors, the concentration of AMH will also decrease. The closer to menopause, AMH will gradually tend to 0.

In the present application, follicle-stimulating hormone (FSH) refers to a hormone secreted by basophilic cells in the anterior lobe of the pituitary gland, the component of which is glycoprotein and the main function of which is to promote follicle maturation. FSH can promote the proliferation and differentiation of follicle granulosa layer cells and promote the growth of the entire ovary. Its action on the testicular seminiferous tubules can promote sperm formation. FSH is secreted in pulses in the human body and changes with the menstrual cycle in women. Measuring FSH in serum is of great significance for understanding the pituitary endocrine function, indirectly understanding the functional status of the ovary, assessing ovarian reserve and ovarian responsiveness, formulating the dosage of ovulation induction drugs, and other diagnosis and treatment of infertility and endocrine diseases.

Recently, serum inhibin B levels have been considered as a marker of follicular development. Inhibin B participates in the selection of follicles in the normal menstrual cycle through endocrine and paracrine effects and promotes follicle growth. One of the functions of inhibin B is to downregulate FSH secretion during the mid-follicular phase of the natural menstrual cycle. It also exerts paracrine effects, stimulating theca cells to produce androgens and LH. The secretion of inhibin B reaches its peak in the early stage of follicles, with a follicle diameter of 10 to 12 mm. Inhibin B on day 5 (early follicular phase) has been shown to be an excellent marker of poor ovarian response and live birth compared with basal markers. Inhibin B is produced primarily by FSH-sensitive follicles, and administration of exogenous FSH causes its increase in growing follicles. Consistent with this, the inventor of the present application found that the dynamic change in inhibin B level ($\Delta$INHB), i.e. the difference between a serum inhibin B concentration on day 6 and an inhibin B concentration on day 2 of menstruation in the ovulation induction cycle, is the best marker for predicting the adjusted dosage of FSH drugs.

Luteinizing hormone (LH) is a glycoprotein gonadotropin secreted by adenohypophysis cells that promotes the conversion of cholesterol into sex hormones in gonad cells. For women, it works with follicle-stimulating hormone (FSH) to promote follicle maturation, estrogen secretion, ovulation, and the formation and maintenance of the corpus luteum, secreting progesterone and estrogen. For men, luteinizing hormone promotes the synthesis and release of testosterone by leydig cells. LH level refers to the LH concentration in venous blood serum sample of a female subject on menstrual days 2 to 4.

Basal E2 level refer to a level of estradiol, which is a steroidal estrogen. There are two types, $\alpha$ and $\beta$. The $\alpha$ type has strong physiological effects. It has strong sex hormone effects, so it is thought that it or its esters are actually the most important sex hormones secreted by the ovaries. The basal estradiol level detected in the present application is the estradiol concentration in venous blood serum sample of a female subject on menstrual days 2 to 4.

BMI is an important standard commonly used internationally to measure the degree of obesity and health of the human body, and is mainly used for statistical analysis. The degree of obesity cannot be judged by the absolute value of body weight, and is naturally related to height. Therefore, BMI obtains a relatively objective parameter through the two values of body weight and height of the human, and uses the range of this parameter to measure body mass. BMI = body weight/height squared (international unit $kg/m^2$).

In the present application, antral follicle count (AFC) refers to the number of all visible follicles with a diameter of 2 to 10 mm in two ovaries on days 2-4 of menstruation. AFC can be measured and counted through ultrasound for follicles.

Ovarian sensitivity is the ratio of the number of mature oocytes (NROs) obtained to the dosage of the exogenous FSH drug.

In order to solve the problem of lack of individualized guidance for predicting the dosage of exogenous FSH drug in the prior art, the present application provides a system for predicting the dosage of an exogenous FSH drug used in a controlled ovarian stimulation cycle when a subject receives ovulation induction treatment by standard GnRH antagonist regimen, which comprises: a data collection module, which is used for acquiring data of age, basal anti-Müllerian hormone (AMH) level, a basal follicle-stimulating hormone (FSH) level or dynamic change in inhibin B level ($\Delta$INHB), and basal antral follicle count (AFC) of the subject; and

a calculation module for obtaining the dosage of an exogenous drug, which is used for performing a first calculation on the data acquired by the data collection module, so as to calculate predicted number of retrieved oocytes (predicted NROs) of the subject in an ovulation induction cycle; performing a second calculation on the data acquired by the data collection module, so as to calculate a ratio of the predicted number of retrieved oocytes (predicted NROs) of the subject in the ovulation induction cycle to the dosage of the exogenous FSH drug administered to the subject in the ovulation induction cycle, i.e., predicted ovarian sensitivity; and calculating the dosage of the exogenous FSH drug to be administered to the subject based on the predicted number of retrieved oocytes (predicted NROs) obtained by the first calculation and the ratio.

In the system of the present application, a model for predicting the number of mature oocytes obtained by a subject during an ovulation induction cycle is firstly established based on several basic indicators, and then a model for predicting ovarian sensitivity is established, so that the prediction of dosage of the exogenous FSH drug can be achieved when there is only one unknown variable, i.e., the dosage of the exogenous FSH drug, in the outcome variable of ovarian sensitivity. The model of the system has $R^2$ of greater than 0.9, which is far better than the model of Lar Marca et al., and is a further optimization of the existing technical model. The system of the present application helps to improve pregnancy outcomes, reduce the incidence of OHSS and lower the cost during COS, and is expected to greatly improve the therapeutic effect of ART.

The subject in the present application is a subject who will receive ovulation induction treatment by standard GnRH antagonist regimen. The number of retrieved oocytes (NROs) of the subject is the number of mature oocytes with a diameter of 10 mm or more, preferably 15 mm or more; wherein the mature oocytes are obtained after the subject firstly receives ovulation induction treatment and the diameter of one or two dominant follicles reaches 18 mm or more during the ovarian stimulation and then the subject undergoes hCG injection for inducing follicle maturation.

In a specific embodiment, the standard GnRH antagonist ovarian stimulation regimen described in the present application is performed as follows: exogenous FSH (human recombinant FSH, referred to as human rFSH) (for example, Gonal-F alfa [Merck Serono, Germany], Puregon beta [MSD, USA], Urofollitropin [Livzon Pharmaceutical Group Inc., China] or Menotrophins [Livzon Pharmaceutical] Group Inc., China]) is administered from the day 2 in the menstrual cycle. The starting dosage of human rFSH is selected based on the age, the basal AMH level, the basal FSH level, the basal AFC level, and BMI. The dosage of rFSH is further adjusted based on the size and number of growing follicles observed by ultrasound and the serum E2 level monitored during ovarian stimulation. When the diameter of the growing follicle reaches 10 to 12 mm, GnRH antagonist treatment is started. When at least two dominant follicles with a diameter exceeding 18mm are observed by ultrasound, hCG (Choriogonadotropin alfa, Merck Serono) is injected at a dosage of 5000 to 10000 IU to trigger final oocyte maturation. Oocytes are recovered 36 to 38 hours after hCG administration. One or two embryos are transferred or embryo cryopreservation is performed. Then progesterone support (progesterone vaginal gel, Merck Serono) in luteal phase is provided to the subject.

In a specific embodiment of the present application, the subject targeted by the system and method involved in the present application is a subject receiving ovulation induction treatment with the standard GnRH antagonist regimen as described above.

Those skilled in the art know that there are many factors that generally affect the retrieved number of oocytes of the subject, such as BMI, duration of infertility, previous in vitro fertilization/intracytoplasmic sperm microinjection-embryo transfer (IVF/ ICSI-ET) number of attempts, the serum basal E2 level, the FSH level and the LH level, the serum AMH level, the left and right ovarian AFCs, first, second, third, fourth and fifth causes of infertility, traditional or minor ovarian stimulation cycle, type of ovarian stimulation/COS regimen, starting dosage and total dosage of rFSH, duration of rFSH treatment (days), name of rFSH, endometrial thickness on human chorionic gonadotropin (hCG) trigger day, etc. In the present application, the inventor of the present application screened various indicators and finally confirmed four important parameters, i.e., the subject's age, basal anti-Müllerian hormone (AMH) level, basal follicle-stimulating hormone (FSH) level and basal antral follicle count (AFC) to calculate the subject's NROs.

In the present application, there are no restrictions on the data collection module, as long as it can be used to acquire the date of the subject's age, basal anti-Miillerian hormone (AMH) level, basal follicle-stimulating hormone (FSH) level or dynamics change in inhibin B levels ($\Delta$INHB) and basal antral follicle count (AFC) are sufficient. Specifically, the basic anti-Müllerian hormone (AMH) level acquired by the data collection module refers to a concentration of an anti-

Müllerian hormone in venous blood of the female subject at any time point during the menstrual period; the basic follicle-stimulating hormone (FSH) level acquired by the data acquisition module refers to a concentration of follicle-stimulating hormone in venous blood of the female subject on the day 2 of menstruation; the basic antral follicle count (AFC) acquired by the data collection module refers to the number of all visible follicles with a diameter of 2 to 10 mm in two ovaries of the female subject on day 2 of menstruation counted by transvaginal B-mode ultrasound; the dynamic change in inhibin B level ($\Delta$INHB) acquired by the data collection module refers to dynamic change in inhibin B level in an early stage of ovulation induction treatment, preferably a difference between a serum inhibin B concentration on day 6 and an inhibin B concentration in venous blood on day 2 of menstruation of the female subject in a cycle of ovulation induction treatment by GnRH antagonist regimen. For the subject who needs to predict the number of retrieved oocytes during ovarian stimulation, the data within the above given period can be used to predict the number of retrieved oocytes based on the system of the present application.

In the present application, the calculation module for obtaining the dosage of an exogenous follicle-stimulating hormone (FSH) drug is used for performing the first calculation and the second calculation on the above data acquired in the data collection module, so as to calculate a ratio of the predicted number of retrieved oocytes (predicted NROs) of the subject in an ovulation induction cycle to the dosage of the exogenous FSH drug to be administered to the subject during the ovulation induction cycle. The system of the present application can predict the recommended initial dosage and adjusted dosage of the exogenous FSH drug administered to the subject during a controlled ovarian stimulation cycle.

In the above system, the brand of the exogenous follicle-stimulating hormone drug is not limited, for example, can be Gonal-F, Puregon, urofollitropin for injection, HMG, etc. The brand of FSH does not affect the accuracy of the system's prediction of initial dosage and adjusted dosage.

In a specific embodiment, the system of the present application is a system for predicting the initial dosage of the exogenous FSH drug to be administered to the subject during a controlled ovarian stimulation cycle, wherein the data collection module is used for acquiring data of the age, the basal anti-Müllerian hormone (AMH) level, the basal follicle-stimulating hormone (FSH) level, and the basal antral follicle count (AFC) of the subject; and the calculation module for obtaining the dosage of the exogenous FSH drug is used for performing a first calculation on the data acquired by the data collection module, so as to calculate predicted number of retrieved oocytes (predicted NROs) of the subject in an ovulation induction cycle; performing a second calculation on the data acquired by the data collection module, so as to calculate a ratio of the predicted retrieved oocytes (predicted NROs) of the subject in the ovulation induction cycle to the dosage of the exogenous FSH drug, i.e., predicted ovarian sensitivity; and calculating the initial dosage of the exogenous FSH drug to be administered to the subject based on the predicted number of retrieved oocytes (predicted NROs) obtained by the first calculation and the ratio obtained by the second calculation.

First of all, it should be understood that data of the age, the basal anti-Müllerian hormone (AMH) level, the basal follicle-stimulating hormone (FSH), the basal antral follicle count (AFC), and the actual detected number of retrieved oocytes (actual number of retrieved oocytes) of a patient who has received ovulation induction treatment by standard GnRH antagonist regimen in an existing database, and formula I for calculating the predicted number of retrieved oocytes (predicted NROs) of the subject, which is obtained by fitting based on the pre-stored data of the patient and negative binomial distribution, are pre-stored in the module. Using the pre-stored formula I, the calculation can be performed for any subject, so as to predict the number of mature oocytes (NROs) obtained by the subject, that is, to obtain the predicted number of retrieved oocytes (predicted NROs).

In addition, it also should be understood that the data of the age, the basal anti-Müllerian hormone (AMH) level, the basal follicle-stimulating hormone (FSH) level, the basal antral follicle count (AFC) of a patient who has received ovulation induction treatment by standard GnRH antagonist regimen in the existing database, and a ratio of the predicted number of retrieved oocytes (predicted NROs) calculated by formula I to an average daily dosage of the exogenous FSH drug used by the patient, and formula II for calculating the predicted ovarian sensitivity of the subject, i.e. the ratio of the predicted number of retrieved oocytes (predicted NROs) to the initial dosage of the exogenous FSH drug to be administrated to the subject, which is obtained by fitting based on the above data, are pre-stored in the module. Using the pre-stored formula II, the calculation can be performed for any subject.

In the present application, the average daily dosage of the exogenous follicle-stimulating hormone drug used by the patient is a ratio of the total dosage of the exogenous FSH drug used by the patient during the ovulation induction treatment with the standard GnRH antagonist regimen to the number of days of using the exogenous FSH drug.

Specifically, the pre-stored formula I is obtained by fitting using the pre-stored data of the age, the basal anti-Müllerian hormone (AMH) level, the basal follicle-stimulating hormone (FSH), the basal antral follicle count (AFC), and the actual detected number of retrieved oocytes of a patient who has received ovulation induction treatment by standard GnRH antagonist regimen in the existing database. The pre-stored formula II is obtained by fitting using the pre-stored data of the age, the basal anti-Müllerian hormone (AMH) level, the basal follicle-stimulating hormone (FSH), and the basal antral follicle count (AFC) of a patient who has received ovulation induction treatment by standard GnRH antagonist regimen in the existing database and the ratio of the predicted number of retrieved oocytes (predicted NROs) calculated by formula I to the average daily dosage of the exogenous FSH drug used by the patient,.

During calculation, the pre-stored formula I is a formula which calculates the predicted number of retrieved oocytes (predicted NROs) of the subject by using the data of the age, the basal anti-Müllerian hormone (AMH) level, the basal follicle-stimulating hormone (FSH) level, and the basal antral follicle count (AFC) of the subject. The pre-stored formula II is a formula which calculates the ratio of the predicted number of retrieved oocytes (predicted NROs) of the subject to the initial dosage of the exogenous FSH drug administered to the subject by using the data of the age, the basal anti-Müllerian hormone (AMH) level, the basal follicle-stimulating hormone (FSH) level, and the basal antral follicle count (AFC) of the subject acquired by the data collection module.

Furthermore, the inventor of the present application constructed the specific formula I for predicting NROs:

$$\text{the predicted NROs} = \mathrm{EXP}(a + b*age* + c*\text{basal FSH} + d*\mathrm{LN}[\text{basal AMH}] + f*\mathrm{LN}[\text{basal AFC}]).$$

Further, in the formula I,

a is any value selected from 1.5576128 to 2.6037078, preferably 2.0806603;
b is any value selected from -0.019097 to 0.0044064, preferably -0.007345;
c is any value selected from -0.045234 to -0.004054, preferably -0.024644;
d is any value selected from 0.348168 to 0.4948875, preferably 0.4215277; and
f is any value selected from 0.0415663 to 0.2566199, preferably 0.1490931.

Based on formula I, the inventor of the present application constructed Formula II for predicting the initial dosage of the exogenous FSH drug:
the predicted ovarian sensitivity of the subject, i.e., the predicted NROs calculated by formula I/ the initial dosage of the exogenous FSH drug= $\mathrm{EXP}(g + h*age + i*\text{basal FSH} + j*\mathrm{LN}[\text{basal AMH}] + k*\text{basal AFC})$.

Further, in the formula II,

g is any value selected from -3.167587 to -2.751518, preferably -2.959552;
h is any value selected from -0.025951 to -0.016355, preferably -0.021153;
i is any value selected from -0.048143 to -0.025727, preferably -0.036935;
j is any value selected from 0.5174476 to 0.6075545, preferably 0.5625011; and
k is any value selected from 0.0241595 to 0.0365579, preferably 0.0303587.

In the above system, the initial dosage of the exogenous FSH drug to be administered to the subject can be calculated by dividing the predicted NROs calculated by formula I by the ratio calculated by formula II.

In the above system, the initial dosage of the exogenous FSH drug to be administered to the subject can be calculated by using formula III, which is:
the initial dosage of the exogenous FSH drug = Round (the predicted NROs calculated by formula I/the predicted ovarian sensitivity calculated by formula II, 0).

In a specific embodiment, the system of the present application is a system for predicting the adjusted dosage of the exogenous FSH drug to be administered to a subject during a controlled ovarian stimulation cycle, wherein the data collection module is used for acquiring data of the age, the basal anti-Müllerian hormone (AMH) level, the basal antral follicle count (AFC) and the dynamic change in inhibin B level (ΔINHB) of the subject; and the calculation module for obtaining the dosage of the exogenous FSH drug is used for performing a first calculation on the data acquired by the data collection module, so as to calculate predicted the numbers of retrieved oocytes (predicted NROs) of the subject in the ovulation induction cycle; performing a second calculation on the data acquired by the data collection module, so as to calculate a ratio of the predicted numbers of retrieved oocytes (predicted NROs) of the subject in the ovulation induction cycle to an adjusted dosage of the exogenous FSH drug, i.e., predicted ovarian sensitivity; and calculating the adjusted dosage of the exogenous FSH drug to be administered to the subject based on the predicted numbers of retrieved oocytes (predicted NROs) obtained by the first calculation and the ratio obtained by the second calculation.

First of all, it should be understood that data of the age, the basal anti-Müllerian hormone (AMH) level, the dynamic change in inhibin B level (ΔINHB), the basal antral follicle count (AFC), and the actual number of retrieved oocytes of a patient who has received ovulation induction treatment by standard GnRH antagonist regimen in the existing database, and the formula IV for calculating the predicted number of retrieved oocytes (predicted NROs) of the subject, which is obtained by fitting based on the pre-stored data of the patient and negative binomial distribution, are pre-stored in the module. Using the pre-stored formula IV, the calculation can be performed for any subject, so as to calculate the predicted number of retrieved oocytes (predicted NROs).

In addition, it should also be understood that the data of the age, the basal anti-Müllerian hormone (AMH) level, the dynamic change in inhibin B level (ΔINHB), and the basal antral follicle count (AFC) of a patient who has received ovulation induction treatment by standard GnRH antagonist regimen in the existing database, and the ratio of the predicted number of retrieved oocytes (predicted NROs) calculated by formula IV to the average daily dosage of the exogenous FSH drug used by the patient, and formula V for calculating the predicted ovarian sensitivity of the subject, i.e., the ratio of the predicted number of retrieved oocytes (predicted NROs) to the adjusted dosage of the exogenous FSH drug to be administrated to the subject, which is obtained by fitting based on the above data, are pre-stored in the module. Using the pre-stored formula V, the calculation can be performed for any subject.

In the present application, the average daily dosage of the exogenous follicle-stimulating hormone drug used by the patient is a ratio of the total dosage of the exogenous FSH drug used by the patient during the ovulation induction treatment with the standard GnRH antagonist regimen to the number of days of using the exogenous FSH drug.

Specifically, the pre-stored formula IV is obtained by fitting using the pre-stored data of the age, the basal anti-Miillerian hormone (AMH) level, the dynamic change in inhibin B level ($\Delta$INHB), the basal antral follicle count (AFC) and the actual number of retrieved oocytes of a patient who has received ovulation induction treatment by standard GnRH antagonist regimen in the existing database. The pre-stored formula V is obtained by fitting using the pre-stored data of the age, the basal anti-Miillerian hormone (AMH) level, the dynamic change in inhibin B level ($\Delta$INHB), and the basal antral follicle count (AFC) of a patient who has received ovulation induction treatment by standard GnRH antagonist regimen in the existing database, and the ratio of the predicted number of retrieved oocytes (predicted NROs) calculated by formula IV to the average daily dosage of the exogenous FSH drug used by the patient.

During calculation, the pre-stored formula IV is a formula which calculates the predicted number of retrieved oocytes (predicted NROs) of the subject by using the data of the age, the basal anti-Müllerian hormone (AMH) level, the dynamic change in inhibin B level ($\Delta$INHB), and the basal antral follicle count (AFC) of the subject acquired by the data collection module. The pre-stored formula V is a formula which calculates the ratio of the predicted number of retrieved oocytes (predicted NROs) of the subject to the adjusted dosage of the exogenous FSH drug administered to the subject by using the data of the age, the basal anti-Miillerian hormone (AMH) level, the dynamic change in inhibin B level ($\Delta$INHB), and the basal antral follicle count (AFC) of the subject acquired by the data collection module.

Furthermore, the inventor of the present application constructed the specific formula IV for predicting NROs:

$$\text{the predicted NROs} = EXP(w + m*age* + n*LN[\text{basal AMH}] + o*LN[\Delta INHB] + p*LN[\text{basal AFC}]).$$

Further, in the formula IV,

w is any value selected from -0.447201 to 0.9161863, preferably 0.2344927;
m is any value selected from -0.017165 to 0.0039328, preferably -0.006616;
n is any value selected from 0.1318094 to 0.3113979, preferably 0.2216036;
o is any value selected from 0.1901643 to 0.3850919, preferably 0.2876281; and
p is any value selected from 0.0541966 to 0.2338079, preferably 0.1440023.

On the basis of formula IV, the inventor of the present application constructed formula V for predicting the adjusted dosage of the exogenous FSH drug, which is:
the predicted ovarian sensitivity of the subject, i.e., the predicted NROs calculated by formula IV/ the adjusted dosage of the exogenous FSH drug = EXP(q + r*age + s*LN[basal AMH] + t*basal AFC + u*LN [$\Delta$INHB]).

Further, in the formula V,

q is any value selected from -5.63461 to -5.108612, preferably -5.371611;
r is any value selected from -0.0264 to -0.015183, preferably -0.020792;
s is any value selected from 0.2696292 to 0.3684551, preferably 0.3190421;
t is any value selected from 0.0273504 to 0.0399372, preferably 0.0336438; and
u is any value selected from 0.3327566 to 0.3940448, preferably 0.3634007.

In the above system, the adjusted dosage of the exogenous FSH drug to be administered to the subject can be calculated by dividing the predicted NROs calculated by formula IV by the ratio calculated by formula V.

In the above system, the adjusted dosage of the exogenous FSH drug to be administered to the subject can be calculated by using formula VI, which is:
the adjusted dosage of the exogenous FSH drug = Round (the predicted NROs calculated by formula IV/the predicted ovarian sensitivity calculated by formula V, 0).

In order to solve the problem of lack of individualized guidance for predicting the dosage of the exogenous FSH drug in the prior art, the present application provides a method for predicting a dosage of an exogenous follicle-stimulating hormone drug to be administered to a subject in a controlled ovarian stimulation cycle, which comprises:

> a data collection step, in which data of age, basal anti-Miillerian hormone (AMH) level, dynamic change in inhibin B level (ΔINHB), and a basal antral follicle count ( AFC) of the subject is acquired; and
>
> a calculation step for an exogenous follicle-stimulating hormone drug dosage, in which first calculation is performed on the data acquired in the data collection step, so as to calculate predicted number of retrieved oocytes (predicted NROs) of the subject in an ovulation induction cycle; second calculation is performed on the data acquired in the data collection step, so as to calculate a ratio of the predicted number of retrieved oocytes (predicted NROs) of the subject in the ovulation induction cycle to an initial dosage of the exogenous FSH drug to be administered to the subject in the ovulation induction cycle, i.e., predicted ovarian sensitivity; and the dosage of the exogenous FSH drug to be administered to the subject is calculated based on the predicted number of retrieved oocytes (predicted NROs) obtained by the first calculation and the ratio.

In the above method, the subject is a subject who will receive standard ovulation induction treatment, and the number of retrieved oocytes (NROs) of the subject is the number of mature oocytes with a diameter of 10 mm or more, preferably 15 mm or more; wherein the mature oocytes are obtained after the subject firstly receives ovulation induction treatment and the diameter of one or two dominant follicles reaches 18 mm or more during the ovarian stimulation and then the subject undergoes hCG injection for inducing follicle maturation.

In the above method, in the data collection step, the basic anti-Müllerian hormone (AMH) level acquired refers to a concentration of anti-Miillerian hormone in venous blood of the subject at any time point during a menstrual period before ovulation induction treatment.

In the above method, in the data collection step, the basal follicle-stimulating hormone (FSH) level acquired refers to a concentration of follicle-stimulating hormone in venous blood of the female subject on day 2 of menstruation before ovulation induction treatment.

In the above method, in the data collection step, the basal antral follicle count (AFC) acquired refers to the number of all visible follicles with a diameter of 2 to 10 mm in two ovaries of the female subject on day 2 of menstruation counted by transvaginal B-mode ultrasound.

In the above method, in the data collection step, the dynamic change in inhibin B level (ΔINHB) acquired refers to a dynamic change in inhibin B level (ΔINHB) in an early stage of ovulation induction treatment, preferably a difference between a serum inhibin B concentration on day 6 and an inhibin B concentration in venous blood on day 2 of menstruation for the female subject in a cycle of ovulation induction treatment by GnRH antagonist regimen.

In the above method, the brand of the exogenous follicle-stimulating hormone drug is not limited, for example, can be Gonal-F, Puregon, urofollitropin for injection, HMG, etc. The brand of FSH does not affect the accuracy of the method's prediction of initial dosage and adjusted dosage.

In a specific embodiment, the method of the present application is a method for predicting the initial dosage of the exogenous FSH drug to be administered to the subject during a controlled ovarian stimulation cycle, wherein in the data collection step of the method, data of the age, the basal anti-Müllerian hormone (AMH) level, the basal follicle-stimulating hormone (FSH) level, and the basal antral follicle count (AFC) of the subject is acquired, and in the calculation step for obtaining the dosage of the exogenous FSH drug of the method, first calculation is performed on the data acquired by the data collection step, so as to calculate predicted number of retrieved oocytes (predicted NROs) of the subject in an ovulation induction cycle; second calculation is performed on the data acquired by the data collection step, so as to calculate a ratio of the predicted number of retrieved oocytes (predicted NROs) of the subject in the ovulation induction cycle to an initial dosage of the exogenous FSH drug, i.e., predicted ovarian sensitivity; and the initial dosage of the exogenous FSH drug to be administered to the subject is calculated based on the predicted number of retrieved oocytes (predicted NROs) obtained by the first calculation and the ratio obtained by the second calculation.

First of all, it should be understood that in the calculation step for obtaining the dosage of the exogenous FSH drug of the method, data of the age, the basal anti-Müllerian hormone (AMH) level, the basal follicle-stimulating hormone (FSH), the basal antral follicle count (AFC), and the actual detected number of retrieved oocytes (actual number of retrieved oocytes) of a patient who has received ovulation induction treatment by standard GnRH antagonist regimen in the existing database, and formula I for calculating the predicted number of retrieved oocytes (predicted NROs) of the subject, which is obtained by fitting based on the pre-stored data of the patient and negative binomial distribution, are pre-stored. Using the pre-stored formula I, the calculation can be performed for any subject, so as to predict the number of mature oocytes (NROs) obtained by the subject, that is, to obtain the predicted number of retrieved oocytes (predicted NROs).

In addition, it also should be understood that in the above method, the data of the age, the basal anti-Müllerian hormone (AMH) level, the basal follicle-stimulating hormone (FSH) level, and the basal antral follicle count (AFC) of a patient who has received ovulation induction treatment by standard GnRH antagonist regimen in the existing database, and a ratio of the predicted number of retrieved oocytes (predicted NROs) calculated by formula I to an average daily dosage of the exogenous FSH drug used by the patient, and formula II for calculating the predicted ovarian sensitivity of the subject, i.e., the ratio of the predicted number of retrieved oocytes (predicted NROs) to the initial dosage of the exogenous FSH drug to be administrated to the subject, which is obtained by fitting based on the above data, are pre-stored in the calculation step for obtaining the dosage of the exogenous FSH drug. Using the pre-stored formula II, the calculation can be performed for any subject.

In the present application, the average daily dosage of the exogenous follicle-stimulating hormone drug used by the patient is a ratio of the total dosage of the exogenous FSH drug used by the patient during the ovulation induction treatment with the standard GnRH antagonist regimen to the number of days of using the exogenous FSH drug.

Specifically, the pre-stored formula I is obtained by fitting using the pre-stored data of the age, the basal anti-Müllerian hormone (AMH) level, the basal follicle-stimulating hormone (FSH), the basal antral follicle count (AFC), and the actual detected number of retrieved oocytes of a patient who has received ovulation induction treatment by standard GnRH antagonist regimen in the existing database. The formula II is obtained by fitting using the pre-stored data of the age, the basal anti-Müllerian hormone (AMH) level, the basal follicle-stimulating hormone (FSH), and the basal antral follicle count (AFC) of a patient who has received ovulation induction treatment by standard GnRH antagonist regimen in the existing database, and the ratio of the predicted number of retrieved oocytes (predicted NROs) calculated by formula I to the average daily dosage of the exogenous FSH drug used by the patient.

During calculation, the pre-stored formula I is a formula which calculates the predicted number of retrieved oocytes (predicted NROs) of the subject by using the data of the age, the basal anti-Müllerian hormone (AMH) level, the basal follicle-stimulating hormone (FSH) level, and the basal antral follicle count (AFC) of the subject acquired in the data collection step. The pre-stored formula II is a formula which calculates the ratio of the predicted number of retrieved oocytes (predicted NROs) of the subject to the initial dosage of the exogenous FSH drug administered to the subject by using the data of the age, the basal anti-Müllerian hormone (AMH) level, the basal follicle-stimulating hormone (FSH) level, and the basal antral follicle count (AFC) of the subject acquired in the data collection step.

In the above method, the formula I is as follows:

$$\text{the predicted NROs} = EXP(a + b * age* + c * \text{basal FSH} + d * LN[\text{basal AMH}] + f * LN[\text{basal AFC}]).$$

Further, in the formula I,

a is any value selected from 1.5576128 to 2.6037078, preferably 2.0806603;
b is any value selected from -0.019097 to 0.0044064, preferably -0.007345;
c is any value selected from -0.045234 to -0.004054, preferably -0.024644;
d is any value selected from 0.348168 to 0.4948875, preferably 0.4215277; and
f is any value selected from 0.0415663 to 0.2566199, preferably 0.1490931.

In the above method, the formula II is as follows:
the predicted ovarian sensitivity of the subject, i.e., the predicted NROs calculated by formula I/ the initial dosage of the exogenous FSH drug= EXP(g + h * age + i * basal FSH + j * LN[basal AMH] + k * basal AFC).

Further, in the formula II,

g is any value selected from -3.167587 to -2.751518, preferably -2.959552;
h is any value selected from -0.025951 to -0.016355, preferably -0.021153;
i is any value selected from -0.048143 to -0.025727, preferably -0.036935;
j is any value selected from 0.5174476 to 0.6075545, preferably 0.5625011; and
k is any value selected from 0.0241595 to 0.0365579, preferably 0.0303587.

In the above method, the initial dosage of the exogenous FSH drug to be administered to the subject can be calculated by dividing the predicted NROs calculated by formula I by the ratio calculated by formula II.

In the above method, the initial dosage of the exogenous FSH drug to be administered to the subject can be calculated by using formula III, which is:
the initial dosage of the exogenous FSH drug = Round (the predicted NROs calculated by formula I/the predicted ovarian sensitivity calculated by formula II, 0)

In a specific embodiment, the method of the present application is a method for predicting the adjusted dosage of the exogenous FSH drug to be administered to a subject during a controlled ovarian stimulation cycle, wherein in the data collection step, data of the age, the basal anti-Müllerian hormone (AMH) level, the basal antral follicle count (AFC), and the dynamic change in inhibin B level (ΔINHB) of the subject is acquired; and in the calculation step for obtaining the dosage of the exogenous FSH drug, first calculation is performed on the data acquired by the data collection step, so as to calculate predicted the number of retrieved oocytes (predicted NROs) of the subject in the ovulation induction cycle; second calculation is performed on the data acquired by the data collection step, so as to calculate a ratio of the predicted number of retrieved oocytes (predicted NROs) of the subject in the ovulation induction cycle to an adjusted dosage of the exogenous FSH drug, i.e., predicted ovarian sensitivity; and the adjusted dosage of the exogenous FSH drug to be administered to the subject is calculated based on the predicted number of retrieved oocytes (predicted NROs) obtained by the first calculation and the ratio obtained by the second calculation.

First of all, it should be understood that in the above method, data of the age, the basal anti-Müllerian hormone (AMH) level, the dynamic change in inhibin B level (△INHB), the basal antral follicle count (AFC), and the actual number of retrieved oocytes of a patient who has received ovulation induction treatment by standard GnRH antagonist regimen in the existing database, and the formula IV for calculating the predicted number of retrieved oocytes (predicted NROs) of the subject, which is obtained by fitting based on the pre-stored data of the patient and negative binomial distribution, are pre-stored. Using the pre-stored formula IV, the calculation can be performed for any subject, so as to calculate the predicted number of retrieved oocytes (predicted NROs).

In addition, it also should be understood that in the above method, the data of the age, the basal anti-Miillerian hormone (AMH) level, the dynamic change in inhibin B level (△INHB), and the basal antral follicle count (AFC) of a patient who has received ovulation induction treatment by standard GnRH antagonist regimen in the existing database, and the ratio of the predicted number of retrieved oocytes (predicted NROs) calculated by formula IV to the average daily dosage of the exogenous FSH drug used by the patient, and formula V for calculating the predicted ovarian sensitivity of the subject, i.e., the ratio of the predicted number of retrieved oocytes (predicted NROs) to the adjusted dosage of the exogenous FSH drug to be administrated to the subject, which is obtained by fitting based on the above data, are pre-stored. Using the pre-stored formula V, the calculation can be performed for any subject.

In the present application, the average daily dosage of the exogenous follicle-stimulating hormone drug used by the patient is a ratio of the total dosage of the exogenous FSH drug used by the patient during the ovulation induction treatment with the standard GnRH antagonist regimen to the number of days of using the exogenous FSH drug.

Specifically, the pre-stored formula IV is obtained by fitting using the pre-stored data of the age, the basal anti-Miillerian hormone (AMH) level, the dynamic change in inhibin B level (△INHB), the basal antral follicle count (AFC) and the actual number of retrieved oocytes of a patient who has received ovulation induction treatment by standard GnRH antagonist regimen in the existing database. The pre-stored formula V is obtained by fitting using the pre-stored data of the age, the basal anti-Miillerian hormone (AMH) level, the dynamic change in inhibin B level (△INHB), and the basal antral follicle count (AFC) of a patient who has received ovulation induction treatment by standard GnRH antagonist regimen in the existing database, and the ratio of the predicted number of retrieved oocytes (predicted NROs) calculated by formula IV to the average daily dosage of the exogenous FSH drug used by the patient.

During calculation, the pre-stored formula IV is a formula which calculates the predicted number of retrieved oocytes (predicted NROs) of the subject by using the data of the age, the basal anti-Müllerian hormone (AMH) level, the dynamic change in inhibin B level (△INHB), and the basal antral follicle count (AFC) of the subject acquired by the data collection step. The pre-stored formula V is a formula which calculates the ratio of the predicted number of retrieved oocytes (predicted NROs) of the subject to the adjusted dosage of the exogenous FSH drug administered to the subject by using the data of the age, the basal anti-Miillerian hormone (AMH) level, the dynamic change in inhibin B level (△INHB), and the basal antral follicle count (AFC) of the subject acquired by the data collection step.

In the above method, the formula IV is as follows: the predicted NROs=EXP(w+m*age*+n*LN[basal AMH]+o*LN[△INHB]+p*LN[basal AFC]).

Further, in the formula IV,

w is any value selected from -0.447201 to 0.9161863, preferably 0.2344927;
m is any value selected from -0.017165 to 0.0039328, preferably -0.006616;
n is any value selected from 0.1318094 to 0.3113979, preferably 0.2216036;
o is any value selected from 0.1901643 to 0.3850919, preferably 0.2876281; and
p is any value selected from 0.0541966 to 0.2338079, preferably 0.1440023.

In the above method, the formula V is as follows:
the predicted ovarian sensitivity of the subject, i.e., the predicted NROs calculated by formula IV/ the adjusted dosage of the exogenous FSH drug = EXP(q+r*age+s*LN[basal AMH]+t*basal AFC+u*LN [△INHB]).

Further, in the formula V,

q is any value selected from -5.63461 to -5.108612, preferably -5.371611;
r is any value selected from -0.0264 to -0.015183, preferably -0.020792;
s is any value selected from 0.2696292 to 0.3684551, preferably 0.3190421;
t is any value selected from 0.0273504 to 0.0399372, preferably 0.0336438; and
u is any value selected from 0.3327566 to 0.3940448, preferably 0.3634007.

In the above method, the adjusted dosage of the exogenous FSH drug to be administered to the subject can be calculated by dividing the predicted NROs calculated by formula IV by the ratio calculated by formula V.

In the above method, the adjusted dosage of the exogenous FSH drug to be administered to the subject can be calculated by using formula VI, which is:

the adjusted dosage of the exogenous FSH drug = Round (the predicted NROs calculated by formula IV/the predicted ovarian sensitivity calculated by formula V, 0).

The initial dosage and adjusted dosage of exogenous FSH predicted by the system or method of the present application are very individualized, and it is impossible for any current exogenous FSH product to achieve such accuracy. Therefore, ART physicians should choose doses close to our recommended doses accordingly. For example, if the predicted starting dosage is 70IU, 75IU could be an alternative.

**Example**

1. Subjects for constructing the model

Data of 669 patients treated at Peking University Third Hospital between April 2020 and September 2020 were collected, and data of 60 patients with incomplete records such as AFC, infertility causes or dosage were further excluded, to construct the model preliminarily. For patients used for preliminary model construction, basic and clinical characteristics of the patient were collected, including last name, medical record number, serial number, age, BMI index, duration of infertility, and previous attempts of in vitro fertilization/ intracytoplasmic sperm microinjection-embryo transfer (IVF/ICSI-ET), the serum basal E2 level, the FSH level and LH level, the serum AMH level, left and right ovarian AFCs, first, second, third, fourth and fifth causes of infertility, traditional or mild ovarian stimulation cycle, type of ovarian stimulation/COS regimen, starting dosage and total dosage of rFSH, duration of rFSH treatment (days), name of rFSH, endometrial thickness on human chorionic gonadotropin (hCG) trigger day, date of oocyte retrieval and NROs.

2. COS treatment

A standard GnRH antagonist ovarian stimulation regimen was performed as follows: human rFSH (e.g., Gonal-F alfa [Merck Serono, Germany], Puregon beta [MSD, USA], Urofollitropin [Livzon Pharmaceutical Group Inc., China] or Menotrophins [Livzon Pharmaceutical Group Inc., China]) was administered from the day 2 in the menstrual cycle. The starting dosage of human rFSH was selected based on the age, the basal AMH level, the basal FSH level, the basal AFC level, BMI, and previous ovarian stimulation result. On the day 6 in the menstrual cycle, the dosage of rFSH was further adjusted based on the size and number of growing follicles observed by ultrasound and the serum E2 level monitored during ovarian stimulation. When the diameter of the growing follicle reached 10 to 12 mm, GnRH antagonist treatment was started.

When at least two dominant follicles with a diameter exceeding 18mm were observed by ultrasound, hCG (Choriogonadotropin alfa, Merck Serono) was injected at a dosage of 5000 to 10000 IU to trigger final oocyte maturation. For high-risk population with ovarian hyperstimulation syndrome, GnRH antagonist can be used alone or combined with 2000 IU of hCG for triggering. Oocytes were recovered 36 to 38 hours after hCG administration. One or two embryos were transferred or embryo cryopreservation was performed. Then progesterone support (progesterone vaginal gel, Merck Serono) in luteal phase was provided to the patient or the subject.

3. Determination of indicators used for model construction

AFC was calculated by measuring follicles with a diameter of 2 to 10 mm in both ovaries on day 2 in the menstrual cycle using transvaginal ultrasound scanning. Venous blood samples were drawn from subjects on day 2 of menstruation using coagulation tubes. Tests on day 2 involved AMH, inhibin B concentration, FSH, LH, E2, testosterone (T), progesterone (P), and androstenedione (A4). Tests on day 6 involved AMH, inhibin B concentration, LH, E2, testosterone (T), progesterone (P), and androstenedione (A4). The serum FSH, LH, E2, P, T and A4 were measured using the Siemens Immulite 2000 immunoassay system (Siemens Healthcare Diagnostics, Shanghai, China).

FSH, LH, E2 and progesterone were measured using the Siemens Immulite 2000 immunoassay system (Siemens Healthcare Diagnostics, Shanghai, PR China). Three-level quality control for FSH, LH and E2 was provided by Bio-RAD Laboratories (Lyphochek Immunoassay Plus Control, three-level, catalog number 370, lot number 40390). Serum AMH concentration and inhibin B concentration were measured using an ultra-sensitive ELISA kit (Ansh Laboratories, Webster, TX, USA), using the quality control provided by the kit. For AMH, inhibin B, FSH, and LH, the coefficient of variation of the three-level or two-level control determined was less than 5%, respectively. For E2, T and A4, the coefficient of variation of the three-level or two-level control determined was less than 10%, respectively. The measurement results are shown in table 1.

Table 1

|  | Level on day 2 | $\Delta$level |
|---|---|---|
| Age (years old) | 32.8±4.3 | NA |
| BMI (kg/m$^2$) | 21.8 (20.0~24.4) | NA |
| FSH (IU/L) | 6.22 (5.14~7.91) | NA |
| AFC | 11.7±6.0 | NA |
| LH (IU/L) | 3.43 (2.44~4.77) | -2.00±2.30 |
| E$_2$ (pmol/L) | 152 (120~177) | 1166 (507~2173) |
| AMH (ng/mL) | 3.03 (1.66~5.33) | -0.77±1.29 |
| inhibin B (pg/mL) | 89 (64~115) | 668 (339~1194) |
| T (nmol/L) | 0.69 (0.69~0.82) | 0.10±0.33 |
| P (nmol/L) | 1.18 (0.95~1.41) | -0.22 (-0.49~0) |
| A4 (nmol/L) | 6.62 (4.99~9.21) | 1.25±3.47 |

Note: Values are expressed as median; $\Delta$level, a dynamic level on day 6 minus that on day 2 of various markers for ovarian reserve; BMI, Body mass index; T, Testosterone; P, Progesterone; A4, Androstenedione (A4); NA, Not applicable.

At the same time, the average daily dosage of exogenous FSH used (the ratio of the total dosage of the exogenous FSH drug used to the number of days of using the exogenous FSH drug), the main cause of infertility, the brand of the exogenous FSH drug and various ovarian stimulation results are shown in Tables 2 to 4 below.

Table 2

| Main cause of infertility | Number of patients |
|---|---|
| Male factor | 180 |
| Endometriosis | 38 |
| Polycystic ovary syndrome | 101 |
| Tubal factor | 166 |
| Unknown reasons and others | 124 |

Table 3

| Brand of the exogenous FSH drug used | Number of patients |
|---|---|
| Gonal-F | 252 |
| Puregon | 85 |
| Urofollitropin | 164 |
| HMG | 10 |
| Gonal-F &HMG | 46 |
| Puregon& HMG | 9 |
| Urofollitropin& HMG | 43 |

Note: Urofollitropin, high-purity urinary follicle-stimulating hormone for injection; HMG, high-purity urinary human menopausal gonadotropins (HMGs) for injection; BMI, Body weight index; T, Testosterone; A4, Androstenedione.

Table 4

| Average daily dosage of exogenous FSH (IU) | 225 (172~300) |
|---|---|
| NROs | 12 (7~17) |
| 2PN fertilization rate | 0.61 (0.44~0.77) |
| Number of embryos that can be transferred per cycle | 3 (2~6) |

Note: PN, pronucleus.

4. Construction of system model for predicting initial dosage

When predicting the initial dosage of the exogenous FSH drug to be administered to the subject during a controlled ovarian stimulation cycle, the present application constructed two models, wherein firstly, model I was constructed to predict the NROs obtained by the subject in an ovulation induction cycle; then model II was constructed based on the NROs predicted by model I, wherein Model II is a model used to obtain the ratio of the predicted number of retrieved oocytes (predicted NROs) of the subject during the ovulation induction cycle to the initial dosage of the exogenous FSH drug to be administered to the subject in the ovulation induction cycle, i.e., a model used to predict the ovarian sensitivity of the subject. The initial variables included in model I and model II are the same, which are cause of infertility, basal FSH, AFC, AMH on the day 2 and day 6, inhibin B, LH, E2, P, testosterone and androstenedione levels.

4.1 Model I

In model I, for the data of the above 609 patients, the distribution of the number of oocytes obtained was first determined. Since the number of retrieved oocytes is count data, poisson distribution or negative binomial distribution may usually be considered. The number of retrieved oocytes of 609 patients in this example is more consistent with the negative binomial distribution. In this example, negative binomial regression was selected to construct statistical model I. The predictive indicators were selected using the pruned forward method and 30% holdback verification. The software JMP Pro v.16 was used to establish the prediction model. The data set of above 609 patients was randomly divided into two parts, one part was used as the training set (426 data, 70%), and the other part was used as the validation set (183 data, 30%).

Firstly, the model was constructed in the training set and the model effect was verified in the validation set. The selection of the prediction model was mainly based on the negative logarithm likelihood value in the validation set. The lower the negative logarithm likelihood value in the validation set, the better the model.

The scaled -Log L($\beta$) no longer decreases when 4 variables are included, thus the 4 variables which are ln[basal AMH], ln[basal AFC], age and basal FSH are finally included in the model according to their importance. At this time, the parameter estimation results of each variable in the prediction model are shown in table 5. Table 5 further shows the 95% confidence interval of each parameter.

Table 5 Parameter estimation results of prediction model I

| Item | Estimated value | Standard deviation | Wald ChiSquare | Probability > ChiSquare | 95% lower limit | 95% upper limit |
|------|-----------------|--------------------|----------------|-------------------------|-----------------|-----------------|
| Intercept | 2.0806603 | 0.2668659 | 60.787787 | <.0001* | 1.5576128 | 26037078 |
| Age | -0.007345 | 0.0059959 | 1.5008009 | 02205 | -0.019097 | 0.0044064 |
| FSH(d2) | -0.024644 | 0.0105053 | 5.5030088 | 0.0190* | -0.045234 | -0.004054 |
| Ln[AMH(d2)] | 0.4215277 | 0.0374292 | 126.83303 | <.0001* | 0.348168 | 0.4948875 |
| Ln[AFC(d2)] | 0.1490931 | 0.0548616 | 7.3854613 | 0.0066* | 0.0415663 | 0.2566199 |

Based on the above method, the following formula I was confirmed in this example:

$$\text{NROs}=\text{EXP}(a+b*age*+c*\text{basal FSH}+d*\text{LN[basal AMH]}+f*\text{LN[basal AFC]});$$

wherein NROs represents the number of mature oocytes; age represents the age of the subject; basal FSH represents the basal follicle-stimulating hormone level of the subject before ovulation induction treatment; basal AMH represents the basal anti-Miillerian hormone level of the subject before ovulation induction treatment; and basal AFC represents the number of all visible follicles with a diameter of 2 to 10 mm in both ovaries on day 2 of menstruation.

In a specific embodiment, AMH refers to a concentration of anti-Müllerian hormone in venous blood of a subject at any time point during the menstrual period before ovulation induction treatment. FSH refers to a concentration of follicle-stimulating hormone in venous blood of the female subject on day 2 of menstruation before ovulation induction treatment. AFC refers to the number of all visible follicles with a diameter of 2 to 10 mm in both ovaries of the female subject on day 2 of menstruation before ovulation induction treatment.

In the above formula I, a is any value selected from 1.5576128 to 2.6037078, preferably 2.0806603;

b is any value selected from -0.019097 to 0.0044064, preferably -0.007345;
c is any value selected from -0.045234 to -0.004054, preferably -0.024644;

d is any value selected from 0.348168 to 0.4948875, preferably 0.4215277; and

f is any value selected from 0.0415663 to 0.2566199, preferably 0.1490931.

The prediction effect of model I constructed using the above method for the training set and validation set is shown in table 6. It can be seen that the above constructed model I achieves good prediction results in both the training set and the verification set, and the predicted data is in high agreement with the actual detected data.

Table 6 Performance of model I on the training set and validation set

| Measure | Training set | Validation set |
|---|---|---|
| -Log possibility | 1266.4585 | 535.1847 |
| BIC | 2569.1154 | 1101.4937 |
| AICc | 2545.1218 | 1082.8578 |
| Generalized R$^2$ | 0.54557 | 0.5322165 |

4.2 Model II

In model II, for the data of the above 609 patients, the outcome variable is ovarian sensitivity, i.e., the ratio of the number of oocytes retrieved (NORs) predicted by model I to the starting dosage of exogenous FSH. According to model I, the predicted NROs were calculated using four basal predictors which were basal AMH, basal AFC, basal FSH and age, and the main effects (contributions) of which were 90.2%, 3.6%, 1.2% and 0.3% respectively.

4.2.1 Normality test of outcome variable

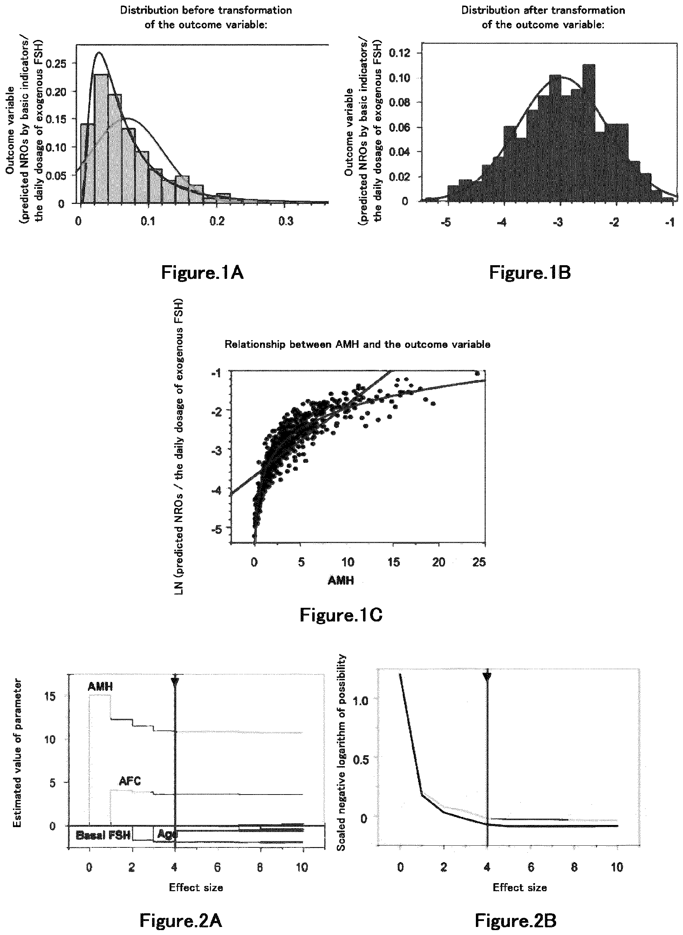

The normality test was performed on the outcome variable, i.e., the ratio of NROs predicted by using the basal predictors to the exogenous FSH. The results show that it is a skewed distribution (Shapiro-Wilk test, W = 0.8691) (FIG. 1A), which is approximately lognormal distribution, so logarithmic transformation is considered. After logarithmic transformation, it is closer to a normal distribution (Shapiro-Wilk test, W = 0.9907) (FIG. 1B). Therefore, in subsequent analyzes, the logarithmic transformation of the outcome variable was used as the dependent variable.

4.2.2 Exploration of linear relationships among main variables

The linear relationship between each independent variable and dependent variable was studied separately. Except for AMH, most variables have a linear relationship. AMH shows a non-linear relationship with result (FIG. 1C). The logarithmic transformation was performed on AMH, and the fitting goodness is significantly better than that before logarithmic transformation, wherein R$^2$ before transformation is 0.6574, and R$^2$ after transformation is 0.8643. Therefore, in subsequent analyses, AMH was analyzed in logarithmic form, and other independent variables were not transformed.

4.2.3 Screening of predictive variables by using lasso regression

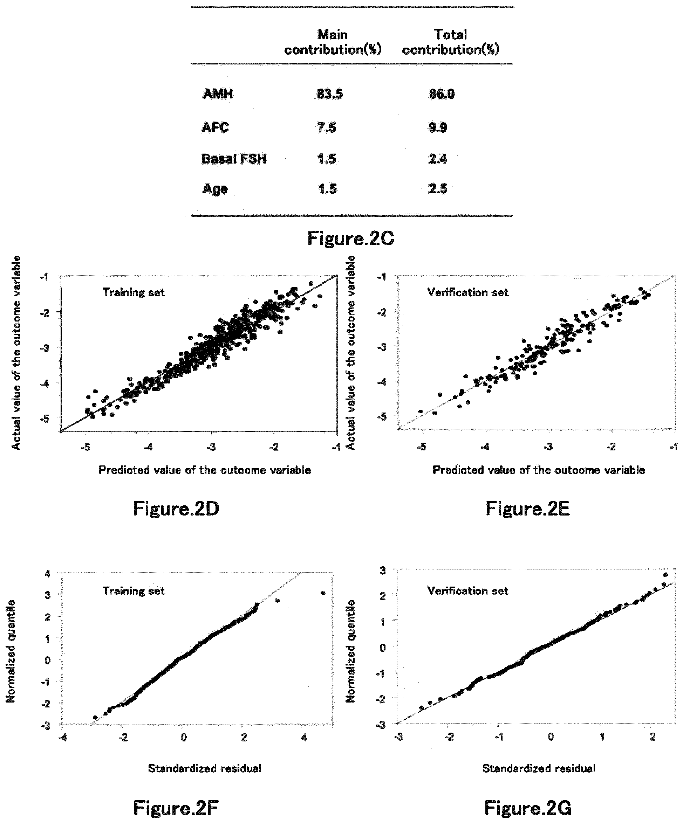

All predictors were screened by using lasso regression. Firstly, the data was randomly divided into the training set and the validation set with a ratio of 0.7:0.3. The best subset method was used for variable selection. The variable screening process is shown in FIGS. 2A and 2B. The scale-LogL(β) value in the validation set no longer decreases when 4 variables are included. The R$^2$ of the model is 0.911 and 0.923 in the training set and validation set respectively. The RMSE of the training set and validation set are 0.237 and 0.224 respectively. The four variables which are log-transformed basal AMH, AFC, basal FSH, and age are finally included in the model for predicting the starting dosage of exogenous FSH, that is, model II. The contributions of the four predictors were evaluated through main effects and total effects respectively, and the results are shown in FIG. 2C, in which AMH has the greatest contribution. At this time, the parameter estimation results of each variable in the prediction model are shown in table 7. Table 7 further shows the 95% confidence interval of each parameter.

Table 7 Parameter estimation results of the prediction model

| Item | Estimated value | Standard deviation | Wald ChiSquare | Probability >ChiSquare | 95% lower limit | 95% upper limit |
|---|---|---|---|---|---|---|
| Intercept | -2.959552 | 0.1061421 | 777.45829 | <.0001* | -3.167587 | -2.751518 |
| Age | -0.021153 | 0.0024481 | 74.661326 | <.0001* | -0.025951 | -0.016355 |

(continued)

| Item | Estimated value | Standard deviation | Wald ChiSquare | Probability >ChiSquare | 95% lower limit | 95% upper limit |
|---|---|---|---|---|---|---|
| Basal FSH | -0.036935 | 0.0057186 | 41.716 | <.0001* | -0.048143 | -0.025727 |
| LN[AMH] | 0.5625011 | 0.0229869 | 598.80802 | <.0001* | 0.5174476 | 0.6075545 |
| AFC | 0.0303587 | 0.0031629 | 92.127956 | <.0001* | 0.0241595 | 0.0365579 |

Based on the above method, the following formula II was confirmed in this example.

The NROs calculated by formula I/ the initial dosage of exogenous follicle-stimulating hormone = (EXP(g+h*age+i*basal FSH+j*LN[basal AMH]+k*basal AFC)

wherein NROs represents the number of mature oocytes; age represents the age of the subject; AFC represents the number of all visible follicles with a diameter of 2 to 10 mm in the two ovaries of the subject on day 2 of menstruation; basal AMH represents the subject's basal anti-Müllerian hormone level before ovulation induction treatment; and basal FSH represents the subject's basal follicle-stimulating hormone level before ovulation induction treatment.

In a specific embodiment, AMH refers to a concentration of anti-Müllerian hormone in venous blood of a subject at any time point during the menstrual period before ovulation induction treatment. AFC refers to the number of all visible follicles with a diameter of 2 to 10 mm in both ovaries of the female subject on day 2 of menstruation before ovulation induction treatment. FSH refers to a concentration of follicle-stimulating hormone in venous blood of the female subject on day 2 of menstruation before ovulation induction treatment.

In the above formula II, g is any value selected from -3.167587 to -2.751518, preferably -2.959552;

h is any value selected from -0.025951 to -0.016355, preferably -0.021153;
i is any value selected from -0.048143 to -0.025727, preferably -0.036935;
j is any value selected from 0.5174476 to 0.6075545, preferably 0.5625011; and
k is any value selected from 0.0241595 to 0.0365579, preferably 0.0303587.

Finally, the initial dosage of the exogenous FSH drug to be administered to the subject was calculated by using formula III, which is:

the initial dosage of the exogenous FSH drug = Round (the predicted NROs calculated by formula I/predicted ovarian sensitivity calculated by formula II, 0).

The effect of model II constructed using the above method for the training set and validation set in predicting the starting dosage of exogenous FSH is shown in table 8 and the scatter plot, which shows the relationship between the predicted outcome variable and the actual outcome variable. If the predictions are completely consistent with the actual results, the scatter points should be completely distributed on the diagonal; as shown in FIGS. 2D and 2E, the points are evenly distributed on both sides of the diagonal, indicating good prediction performance. The residual plot is also used to estimate the effect. For the ideal fitting, the points should be evenly distributed on the diagonal; as shown in FIGS. 2F and 2G, the points are also evenly distributed on both sides of the diagonal in the residual plot, with a normal distribution and very small prediction deviation. All these results indicate that model II has good prediction performance.

Table 8 Performance of model II on the training set and validation set

| Measure | Training set | Validation set |
|---|---|---|
| BIC | 17.757448 | 4.4399565 |
| AICc | -6.251061 | -14.23224 |
| Generalized $R^2$ | 0.9111004 | 0.922593 |

5 Construction of system model for predicting adjusted dosage

When predicting the adjusted dosage of the exogenous FSH drug to be administrated to the subject during a controlled ovarian stimulation cycle, the present application constructed two models wherein firstly, model III was constructed to predict the NROs obtained by the subject in an ovulation induction cycle; then model IV was constructed based on the NROs predicted by model III, wherein model IV is a model used to obtain the ratio of the predicted number of retrieved oocytes (predicted NROs) of the subject during the ovulation induction cycle to the adjusted dosage of the exogenous FSH drug to be administrated to the subject in the ovulation induction cycle, i.e., a model used to predict the ovarian sensitivity of the subject. The initial variables included in model III and model VI are the same, which are age, BMI, cause of infertility, basal FSH, AFC, AMH on the day 2 and day 6, inhibin B, LH, E2, P, testosterone and androstenedione levels.

5.1 Model III

In model III, for the data of the above 609 patients, the distribution of the number of retrieved oocytes was first determined. Since the number of retrieved oocytes is count data, poisson distribution or negative binomial distribution may usually be considered. The number of retrieved oocytes of 609 patients in this example is more consistent with the negative binomial distribution. In this example, negative binomial regression was selected to construct statistical model III. The predictive indicators were selected using the pruned forward method and 30% holdback verification. The software JMP Pro v.16 was used to establish the prediction model. The set of data of above 609 patients was randomly divided into two parts, one part was used as the training set (426 data, 70%), and the other part was used as the validation set (183 data, 30%).

Firstly, the model was constructed in the training set and the model effect was verified in the validation set. The selection of the prediction model was mainly based on the negative logarithm likelihood value in the validation set. The lower the negative logarithm likelihood value in the validation set, the better the model.

The scaled -Log L($\beta$) no longer decreases when 4 variables are included, thus the 4 variables which are ln[$\Delta$INHB], ln[basal AMH], AFC, and age are finally included in the model according to their importance. At this time, the parameter estimation results of each variable in the prediction model are shown in table 9. Table 9 further shows the 95% confidence interval of each parameter.

Table 9 Parameter estimation results of prediction model III

| Item | Estimated value | Standard deviation | Wald ChiSquare | Probability> ChiSquare | 95% lower limit | 95% upper limit |
|---|---|---|---|---|---|---|
| Intercept | 0.2344927 | 0.3478093 | 0.4545444 | 0.5002 | -0.447201 | 0.9161863 |
| Age | -0.006616 | 0.0053823 | 1.5111152 | 0.2190 | -0.017165 | 0.0039328 |
| Ln[AFC(d2)] | 0.1440023 | 0.0458201 | 9.8770549 | 0.0017* | 0.0541966 | 0.2338079 |
| Ln[INHB(d6-2)] | 0.2876281 | 0.0497274 | 33.455834 | <.0001* | 0.1901643 | 0.3850919 |
| Ln[AMH(d2)] | 0.2216036 | 0.0458142 | 23.396603 | <.0001* | 0.1318094 | 03113979 |

Based on the above method, the following formula IV was confirmed in this example:

the predicted NROs=EXP(w+m*age*+n*LN[basal AMH]+o*LN[$\Delta$INHB]+p*LN[basal AFC]);

wherein NROs represents the number of mature oocytes; age represents the age of the subject; $\Delta$INHB represents dynamic change in inhibin B level of the subject in an early stage of ovulation induction treatment; basal AMH represents the basal anti-Miillerian hormone level of the subject before ovulation induction treatment; and basal AFC represents the number of all visible follicles with a diameter of 2 to 10 mm in both ovaries on day 2 of menstruation.

In a specific embodiment, AMH refers to a concentration of anti-Müllerian hormone in venous blood of the subject at any time point during the menstrual period before ovulation induction treatment. $\Delta$INHB refers to a difference between a serum inhibin B concentration of the female subject on day 6 of menstruation and an inhibin B concentration in venous blood of the female subject on day 2 of menstruation during ovulation induction treatment by GnRH antagonist regimen. AFC refers to the number of all visible follicles with a diameter of 2 to 10 mm in both ovaries of the female subject on day 2 of menstruation before ovulation induction treatment.

In the above formula IV, w is any value selected from -0.447201 to 0.9161863, preferably 0.2344927;

m is any value selected from -0.017165 to 0.0039328, preferably -0.006616;
n is any value selected from 0.1318094 to 0.3113979, preferably 0.2216036;
o is any value selected from 0.1901643 to 0.3850919, preferably 0.2876281; and
p is any value selected from 0.0541966 to 0.2338079, preferably 0.1440023.

The prediction effect of model III constructed using the above method for the training set and validation set is shown in table 10. It can be seen that the above constructed model III achieves good prediction results in both the training set and the verification set, and the predicted data is in high agreement with the actual detected data.

Table 10 Performance of model III on the training set and validation set

| Measure | Training set | Validation set |
|---|---|---|
| -Log possibility | 1214.7173 | 533.08453 |
| BIC | 2465.6761 | 1097.36 |
| AICc | 2441.638 | 1078.6518 |
| Generalized $R^2$ | 0.642557 | 0.6160343 |

5.2 Model IV

In model IV, for the data of the above 609 patients, the outcome variable is ovarian sensitivity, i.e., the ratio of the number of oocytes retrieved (NORs) predicted by model I to the starting dosage of exogenous FSH. According to model III, the predicted NROs were calculated using four basal predictors which were dynamic change in inhibin B level (day 6 minus day 2), basal AMH, basal AFC, and age, the main effects (contributions) of which were 48.9%, 30.0%, 12.7% and 2.0%, and the total effects (contributions) of which were 50.8%, 31.8%, 14.5% and 3.2% respectively.

5.2.1 Normality test of outcome variable

The normality test was performed on the outcome variable, i.e., the ratio of NROs predicted by using the basal predictors to the exogenous FSH. The results show that it is a skewed distribution (Shapiro-Wilk test, W = 0.8522), which is approximately lognormal distribution, so logarithmic transformation is considered. After logarithmic transformation, it is closer to a normal distribution (Shapiro-Wilk test, W = 0.9916). Therefore, in subsequent analyzes, the logarithmic transformation of the outcome variable was used as the dependent variable.

5.2.2 Exploration of linear relationships among main variables

The linear relationship between each independent variable and dependent variable was studied separately. Most variables have a linear relationship, and the AMH, inhibin B level, and dynamic change in inhibin B level have an obvious nonlinear relationship with the results. The logarithmic transformation was performed on these three variables, and the fitting goodness is significantly better than that before logarithmic transformation. For AMH, $R^2$ before transformation is 0.5829 , and $R^2$ after transformation is 0.7904; for the inhibin B level, $R^2$ before transformation is 0.2255, and $R^2$ after transformation is 0.2608; for the dynamic change in inhibin B level, $R^2$ before transformation is 0.5613, and $R^2$ after transformation is 0.7247. Therefore, in subsequent analyses, AMH, the inhibin B level, and the dynamic change in inhibin B level were analyzed in logarithmic form, and other independent variables were not transformed.

5.2.3 Screening of predictive variables by using lasso regression

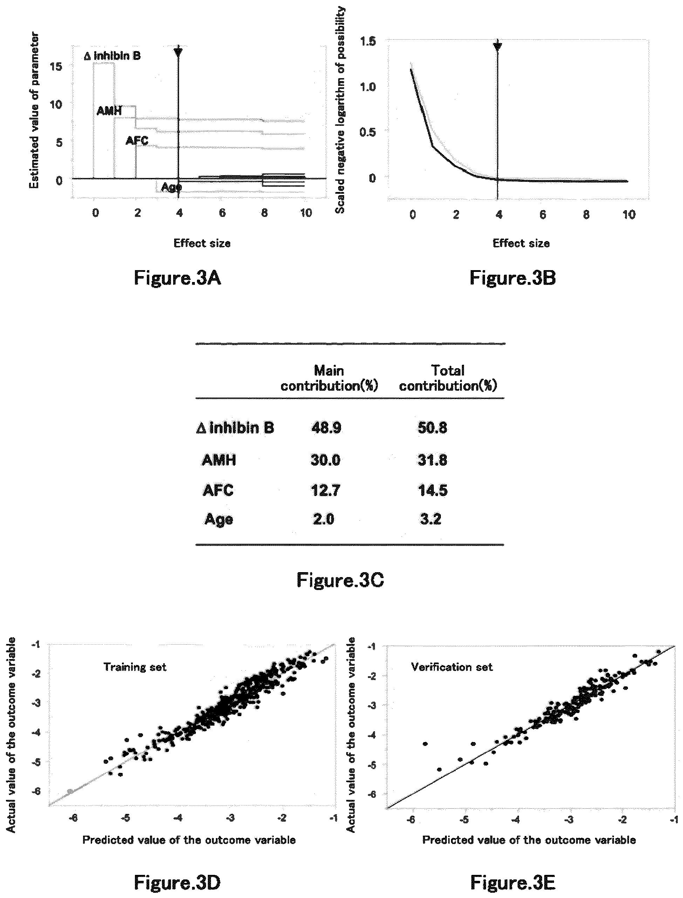

All predictors were screened by using lasso regression. Firstly, the data was randomly divided into the training set and the validation set with a ratio of 0.7:0.3. The best subset method was used for variable selection. The variable screening process is shown in FIGS. 3A and 3B. The scale-LogL($\beta$) value in the validation set no longer decreases when 4 variables are included. The $R^2$ of the model is 0.922 and 0.909 in the training set and validation set respectively. The RMSE of the training set and validation set are 0.236 and 0.231 respectively. The four variables which are log-transformed basal AMH, log-transformed $\Delta$INHB, AFC, and age are finally included in the model for predicting the adjusted dosage of exogenous FSH, that is, model IV. The contributions of the four predictors were evaluated through main effects and total effects respectively, and the results are shown in FIG. 3C, in which $\Delta$INHB has the greatest contribution. At this time, the parameter estimation results of each variable in the prediction model are shown in table 11. Table 11 further shows the 95% confidence interval of each parameter.

Table 11 Parameter estimation results of the prediction model

| Item | Estimated value | Standard deviation | Wald ChiSquare | Probability >C hiSquare | 95% lower limit | 95% upper limit |
|---|---|---|---|---|---|---|
| Intercept | -5.371611 | 0.1341857 | 1602.494 | <.0001* | -5.63461 | -5.108612 |
| Age | -0.020792 | 0.0028614 | 52.797412 | <.0001* | -0.0264 | -0.015183 |
| Ln[AMH] | 0.3190421 | 0.0252111 | 160.14418 | <.0001* | 0.2696292 | 0.3684551 |
| AFC | 0.0336438 | 0.003211 | 109.78355 | <.0001* | 0.0273504 | 0.0399372 |

(continued)

| Item | Estimated value | Standard deviation | Wald ChiSquare | Probability >C hiSquare | 95% lower limit | 95% upper limit |
|---|---|---|---|---|---|---|
| Ln[INHB(d6-d2)] | 0.3634007 | 0.015635 | 540.22465 | <.0001* | 0.3327566 | 0.3940448 |

Based on the above method, the following formula V was confirmed in this example.

The NROs calculated by formula V/ the adjusted dosage of exogenous follicle-stimulating hormone = EXP(q+r*Age+s*LN[Basal AMH]+t*Basal AFC+u*LN[ΔINHB])

wherein NROs represents the number of mature oocytes; Age represents the age of the subject; AFC represents the number of all visible follicles with a diameter of 2 to 10 mm in the two ovaries of the subject on day 2 of menstruation; Basal AMH represents the basal anti-Müllerian hormone level of the subject before ovulation induction treatment; and ΔINHB represents the dynamic change in inhibin B level of the subject in the early stage during ovulation induction treatment.

In a specific embodiment, AMH refers to a concentration of the anti-Müllerian hormone in the blood of a subject at any time point during the menstrual period before ovulation induction treatment. AFC refers to the number of all visible follicles with a diameter of 2 to 10 mm in both ovaries of the female subject on day 2 of menstruation before ovulation induction treatment. ΔINHB refers to a difference between a serum inhibin B concentration of the female subject on day 6 of menstruation and an inhibin B concentration in venous blood of the female subject on day 2 of menstruation during ovulation induction treatment by GnRH antagonist regimen.

In the above formula V, q is any value selected from -5.63461 to -5.108612, preferably -5.371611;

r is any value selected from -0.0264 to -0.015183, preferably -0.020792;
s is any value selected from 0.2696292 to 0.3684551, preferably 0.3190421;
t is any value selected from 0.0273504 to 0.0399372, preferably 0.0336438; and
u is any value selected from 0.3327566 to 0.3940448, preferably 0.3634007.

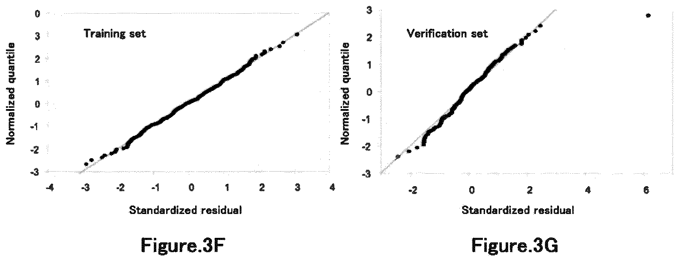

The effect of model IV constructed using the above method on the training set and validation set in predicting the adjusted dosage of exogenous FSH is shown in table 12 and the scatter plot, which shows the relationship between the predicted outcome variable and the actual outcome variable. If the predictions are completely consistent with the actual results, the scatter points should be completely distributed on the diagonal; as shown in FIGS. 3D and 3E, the scatter points are evenly distributed on both sides of the diagonal, indicating good prediction performance. The residual plot is also used to estimate the effect. For the ideal fitting, the points should be evenly distributed on the diagonal; as shown in FIGS. 3F and 3G, the scatter points are also evenly distributed on both sides of the diagonal in the residual plot, with a normal distribution and very small prediction deviation. All these results indicate that model IV has good prediction performance.

Table 12 Performance of model IV on the training set and validation set

| Measure | Training set | Validation set |
|---|---|---|
| -Log possibility | -10.77973 | -7.957709 |
| BIC | 14.624653 | 15.208896 |
| AICc | -9.35408 | -3.427047 |
| Generalized R$^2$ | 0.9219982 | 0.908807 |

Although the embodiments of the present application have been described above in conjunction with the accompanying drawings, the present application is not limited to the above-mentioned specific embodiments and application fields. The above-mentioned specific embodiments are only illustrative and instructive, rather than restrictive. Under the inspiration of this description and without departing from the scope of protection of the claims of the present application, those of ordinary skill in the art can also make many forms, which all belong to the scope of protection of the present application.

## Claims

1. A system for predicting a dosage of an exogenous follicle-stimulating hormone (FSH) drug to be administered to a subject in a controlled ovarian stimulation cycle, comprising:

a data collection module, which is used for acquiring data of age, basal anti-Müllerian hormone (AMH) level, basal follicle-stimulating hormone (FSH) level or dynamic change in inhibin B level (ΔINHB), and basal antral follicle count (AFC) of the subject; and

a calculation module for obtaining the dosage of an exogenous follicle-stimulating hormone (FSH) drug, which is used for performing a first calculation on the data acquired by the data collection module, so as to calculate predicted number of retrieved oocytes (predicted NROs) of the subject in an ovulation induction cycle; performing a second calculation on the data acquired by the data collection module, so as to calculate a ratio of the predicted number of retrieved oocytes (predicted NROs) of the subject in the ovulation induction cycle to the dosage of the exogenous FSH drug, i.e., predicted ovarian sensitivity; and calculating the dosage of the exogenous FSH drug to be administered to the subject based on the predicted NROs obtained by the first calculation and the ratio obtained by the second calculation.

2. The system according to claim 1, wherein

the subject is a subject who will receive standard ovulation induction treatment, and the number of retrieved oocytes (NROs) of the subject is the number of mature oocytes with a diameter of 10 mm or more, preferably 15 mm or more; wherein the mature oocytes are obtained after the subject firstly receives ovulation induction treatment and the diameter of one or two dominant follicles reaches 18 mm or more during the ovarian stimulation and then the subject undergoes hCG injection for inducing follicle maturation.

3. The system according to claim 1 or 2, wherein

in the data collection module, the basal anti-Müllerian hormone (AMH) level acquired refers to a concentration of anti-Müllerian hormone in venous blood of the subject at any time point during a menstrual period before ovulation induction treatment.

4. The system according to any one of claims 1 to 3, wherein

in the data collection module, the basal follicle-stimulating hormone (FSH) level acquired refers to a concentration of follicle-stimulating hormone in venous blood of the female subject on day 2 to day 4 of menstruation before ovulation induction treatment.

5. The system according to any one of claims 1 to 4, wherein

in the data collection module, the basal antral follicle count (AFC) acquired refers to the number of all visible follicles with a diameter of 2 to 10 mm in two ovaries of the female subject on day 2 of menstruation counted by transvaginal B-mode ultrasound.

6. The system according to any one of claims 1 to 5, wherein

in the data collection module, the dynamic change in inhibin B level (ΔINHB) acquired refers to a dynamic change in inhibin B level (ΔINHB) in an early stage of ovulation induction treatment, preferably a difference between an serum inhibin B concentration on day 6 of menstruation and an inhibin B concentration in venous blood on day 2 of menstruation of the female subject in a cycle of ovulation induction treatment by GnRH antagonist regimen.

7. The system according to any one of claims 1 to 6, wherein

the data collection module is used for acquiring data of the age, the basal anti-Miillerian hormone (AMH) level, the basal follicle-stimulating hormone (FSH) level, and the basal antral follicle count (AFC) of the subject; and

the calculation module for obtaining the dosage of the exogenous follicle-stimulating hormone (FSH) drug is used for performing a first calculation on the data acquired by the data collection module, so as to calculate predicted the number of retrieved oocytes (predicted NROs) of the subject in the ovulation induction cycle; performing a second calculation on the data acquired by the data collection module, so as to calculate a ratio of the predicted number of retrieved oocytes (predicted NROs) of the subject in the ovulation induction cycle to an initial dosage of the exogenous FSH drug, i.e., predicted ovarian sensitivity; and calculating the initial dosage of the exogenous FSH drug to be administered to the subject based on the predicted number of retrieved oocytes (predicted NROs) obtained by the first calculation and the ratio obtained by the second calculation.

8. The system according to claim 7, wherein

in the calculation module for obtaining the dosage of the exogenous FSH drug, formula I for calculating the predicted number of retrieved oocytes (predicted NROs) of the subject is pre-stored, wherein the formula I is obtained by fitting based on data of the age, the basal anti-Miillerian hormone (AMH) level, the basal follicle-stimulating hormone (FSH), the basal antral follicle count (AFC) and the actual number of retrieved oocytes of a patient who has received ovulation induction treatment by standard GnRH antagonist regimen in an existing database.

9. The system according to claim 8, wherein

in the calculation module for obtaining the dosage of the exogenous FSH drug, the formula I is a calculation formula I obtained by using negative binomial distribution of the outcome variable to fit the data of the age, the basal anti-Miillerian hormone (AMH) level, the basal follicle-stimulating hormone (FSH), the basal antral follicle count (AFC) and the actual number of retrieved oocytes (outcome variable) of the patient who has received ovulation induction treatment by standard GnRH antagonist regimen in the existing database; and

the formula I can be used to calculate the predicted number of retrieved oocytes (predicted NROs) of the subject by using the data of the age, the basal anti-Miillerian hormone (AMH) level, the basal follicle-stimulating hormone (FSH) level, and the basal antral follicle count (AFC) of the subject acquired by the data collection module.

10. The system according to claim 9, wherein the formula I is:

$$\text{the predicted NROs} = EXP(a+b*age*+c*basal\ FSH+d*LN[basal\ AMH]+f*LN[basal\ AFC]);$$

wherein a is any value selected from 1.5576128 to 2.6037078, preferably 2.0806603;
b is any value selected from -0.019097 to 0.0044064, preferably -0.007345;
c is any value selected from -0.045234 to -0.004054, preferably -0.024644;
d is any value selected from 0.348168 to 0.4948875, preferably 0.4215277; and
f is any value selected from 0.0415663 to 0.2566199, preferably 0.1490931.

11. The system according to claim 10, wherein

in the calculation module for obtaining the dosage of the exogenous FSH drug, formula II for calculating the predicted ovarian sensitivity of the subject, i.e., the ratio of the predicted number of retrieved oocytes (predicted NROs) to the initial dosage of the exogenous FSH drug is pre-stored, wherein the formula II is obtained by fitting based on the data of the age, the basal anti-Müllerian hormone (AMH) level, the basal follicle-stimulating hormone (FSH) , and the basal antral follicle count (AFC) of the patient who has received ovulation induction treatment by standard GnRH antagonist regimen in the existing database, and a ratio of the predicted number of retrieved oocytes (predicted NROs) calculated by formula I to an average daily dosage of the exogenous FSH drug used by the patient;

wherein the average daily dosage of the exogenous FSH drug used by the patient refers to a ratio of a total dosage of the exogenous FSH drug used by the patient to the number of days of using the exogenous FSH drug during the past ovulation induction treatment by standard GnRH antagonist regimen.

12. The system according to claim 11, wherein
in the calculation module for obtaining the dosage of the exogenous FSH drug, the formula II can be used to calculate the predicted ovarian sensitivity of the subject, i.e., the ratio of the predicted numbers of retrieved oocytes (predicted NROs) to the initial dosage of the exogenous FSH drug to be administrated to the subject, by using the data of the age, the basal anti-Müllerian hormone (AMH) level, the basal follicle-stimulating hormone (FSH) level, and the basal antral follicle count (AFC) of the subject acquired by the data collection module.

13. The system according to claim 11 or 12, wherein the formula II is:

the predicted ovarian sensitivity of the subject, i.e., the predicted NROs calculated by formula I/ the initial dosage of the exogenous FSH drug= EXP(g+h*age+i*basal FSH+j*LN[basal AMH]+k*basal AFC)
wherein g is any value selected from -3.167587 to -2.751518, preferably -2.959552;
h is any value selected from -0.025951 to -0.016355, preferably -0.021153;
i is any value selected from -0.048143 to -0.025727, preferably -0.036935;
j is any value selected from 0.5174476 to 0.6075545, preferably 0.5625011; and
k is any value selected from 0.0241595 to 0.0365579, preferably 0.0303587.

14. The system according to claim 13, wherein based on the predicted numbers of retrieved oocytes (predicted NROs) obtained by the first calculation and the ratio obtained by the second calculation, the initial dosage of the exogenous FSH drug to be administered to the subject is calculated by using formula III, which is:

the initial dosage of the exogenous FSH drug = Round (the predicted NROs calculated by formula I/the predicted ovarian sensitivity calculated by formula II, 0).

**15.** The system according to any one of claims 1 to 6, wherein

the data collection module is used for acquiring data of the age, the basal anti-Miillerian hormone (AMH) level, the basal antral follicle count (AFC) and the dynamic change in inhibin B level of the subject; and
the calculation module for obtaining the dosage of the exogenous follicle-stimulating hormone (FSH) drug is used for performing a first calculation on the data acquired by the data collection module, so as to calculate the predicted numbers of retrieved oocytes (predicted NROs) of the subject in the ovulation induction cycle; performing a second calculation on the data acquired by the data collection module, so as to calculate a ratio of the predicted number of retrieved oocytes (predicted NROs) of the subject in the ovulation induction cycle to an adjusted dosage of the exogenous FSH drug, i.e., predicted ovarian sensitivity; and calculating the adjusted dosage of the exogenous FSH drug to be administered to the subject based on the predicted NROs obtained by the first calculation and the ratio obtained by the second calculation.

**16.** The system according to claim 15, wherein
in the calculation module for obtaining the dosage of the exogenous FSH drug, formula IV for calculating the predicted number of retrieved oocytes (predicted NROs) of the subject is pre-stored, wherein the formula IV is obtained by fitting based on the data of the age, the basal anti-Miillerian hormone (AMH) level, the basal antral follicle count (AFC), the dynamic change in inhibin B level (ΔINHB), and the actual number of retrieved oocytes of the patient who has received ovulation induction treatment by standard GnRH antagonist regimen in the existing database.

**17.** The system according to claim 16, wherein

in the calculation module for obtaining the dosage of the exogenous FSH drug, the formula IV is a calculation formula IV obtained by using negative binomial distribution of the outcome variable to fit the data of the age, the basal anti-Müllerian hormone (AMH) level, the basal antral follicle count (AFC), the dynamic change in inhibin B level (ΔINHB), and the actual number of retrieved oocytes (outcome variable) of the patient who has received ovulation induction treatment by standard GnRH antagonist regimen in the existing database; and
the formula IV can be used to calculate the predicted number of retrieved oocytes (predicted NROs) of the subject by using the data of the age, the basal anti-Miillerian hormone (AMH) level, the basal antral follicle count (AFC) and the dynamic change in inhibin B level (ΔINHB) of the subject acquired by the data collection module.

**18.** The system according to claim 17, wherein the formula IV is:

the predicted NROs=EXP(w+m*age*+n*LN[basal AMH]+o*LN[ΔINHB]+p*LN[basal AFC]);

wherein w is any value selected from -0.447201 to 0.9161863, preferably 0.2344927;
m is any value selected from -0.017165 to 0.0039328, preferably -0.006616;
n is any value selected from 0.1318094 to 0.3113979, preferably 0.2216036;
o is any value selected from 0.1901643 to 0.3850919, preferably 0.2876281; and
p is any value selected from 0.0541966 to 0.2338079, preferably 0.1440023.

**19.** The system according to claim 18, wherein,

in the calculation module for obtaining the dosage of the exogenous FSH drug, formula V for calculating the predicted ovarian sensitivity of the subject, i.e., the ratio of the predicted number of retrieved oocytes (predicted NROs) to the adjusted dosage of the exogenous FSH drug, is pre-stored; wherein the formula V is obtained by fitting based on the data of the age, the basal anti-Miillerian hormone (AMH) level, the basal antral follicle count (AFC), and the dynamic change in inhibin B level (ΔINHB) of the patient who has received ovulation induction treatment by standard GnRH antagonist regimen in the existing database, and a ratio of the predicted number of retrieved oocytes (predicted NROs) calculated by formula IV to an average daily dosage of the exogenous FSH drug used by the patient,
wherein the average daily dosage of the exogenous FSH drug used by the patient refers to a ratio of a total dosage of the exogenous FSH drug used by the patient to the number of days of using the exogenous FSH drug during the past ovulation induction treatment by standard GnRH antagonist regimen.

20. The system according to claim 19, wherein
in the calculation module for obtaining the dosage of the exogenous FSH drug, the formula V can be used to calculate the predicted ovarian sensitivity of the subject, i.e., the ratio of the predicted number of retrieved oocytes (predicted NROs) to the adjusted dosage of the exogenous FSH drug to be administrated to the subject, by using the data of the age, the basal anti-Müllerian hormone (AMH) level, the basal antral follicle count (AFC) and the dynamic change in inhibin B level (ΔINHB) of the subject acquired by the data collection module.

21. The system according to claim 19 or 20, wherein the formula V is:

the predicted ovarian sensitivity of the subject, i.e., the predicted NROs calculated by formula IV/ the adjusted dosage of the exogenous FSH drug = EXP(q+r*age+s*LN[basal AMH]+t*basal AFC+u*LN[ΔINHB]),
wherein q is any value selected from -5.63461 to -5.108612, preferably -5.371611;
r is any value selected from -0.0264 to -0.015183, preferably -0.020792;
s is any value selected from 0.2696292 to 0.3684551, preferably 0.3190421;
t is any value selected from 0.0273504 to 0.0399372, preferably 0.0336438; and
u is any value selected from 0.3327566 to 0.3940448, preferably 0.3634007.

22. The system according to claim 21, wherein based on the predicted number of retrieved oocytes (predicted NROs) obtained by the first calculation and the ratio obtained by the second calculation, the adjusted dosage of the exogenous FSH drug to be administered to the subject is calculated by using Formula VI which is:
the adjusted dosage of the exogenous FSH drug = Round (the predicted NROs calculated by formula VI/the predicted ovarian sensitivity calculated by formula V, 0).

23. A method for predicting a dosage of an exogenous FSH drug to be administered to a subject in a controlled ovarian stimulation cycle, comprising:

a data collection step, in which data of age, basal anti-Müllerian hormone (AMH) level, basal follicle-stimulating hormone (FSH) level or dynamic change in inhibin B level (ΔINHB), and basal antral follicle count (AFC) of the subject is acquired; and
a calculation step for obtaining the dosage of an exogenous FSH drug, in which a first calculation is performed on the data acquired in the data collection step, so as to calculate predicted number of retrieved oocytes (predicted NROs) of the subject in an ovulation induction cycle; a second calculation is performed on the data acquired in the data collection step, so as to calculate a ratio of the predicted number of retrieved oocytes (predicted NROs) of the subject in the ovulation induction cycle to the dosage of the exogenous FSH drug, i.e., predicted ovarian sensitivity; and the dosage of the exogenous FSH drug to be administered to the subject is calculated based on the predicted number of retrieved oocytes (predicted NROs) obtained by the first calculation and the ratio obtained by the second calculation.

24. The method according to claim 23, wherein
the subject is a subject who will receive standard ovulation induction treatment, and the number of retrieved oocytes (NROs) of the subject is the number of mature oocytes with a diameter of 10 mm or more, preferably 15 mm or more; wherein the mature oocytes are obtained after the subject firstly receives ovulation induction treatment and the diameter of one or two dominant follicles reaches 18 mm or more during the ovarian stimulation and then the subject undergoes hCG injection for inducing follicle maturation.

25. The method according to claim 23 or 24, wherein
in the data collection step, the basal anti-Müllerian hormone (AMH) level acquired refers to a concentration of anti-Müllerian hormone in venous blood of the subject at any time point during a menstrual period before ovulation induction treatment.

26. The method according to any one of claims 23 to 25, wherein
in the data collection step, the basal follicle-stimulating hormone (FSH) level acquired refers to a concentration of follicle-stimulating hormone in venous blood of the female subject on day 2 to day 4 of menstruation before ovulation induction treatment.

27. The method according to any one of claims 23 to 26, wherein

in the data collection step, the basal antral follicle count (AFC) acquired refers to the number of all visible follicles with a diameter of 2 to 10 mm in two ovaries of the female subject on day 2 of menstruation counted by transvaginal B-mode ultrasound.

28. The method according to any one of claims 23 to 27, wherein
in the data collection step, the dynamic change in inhibin B level ($\Delta$INHB) acquired refers to a dynamic change in inhibin B level ($\Delta$INHB) in an early stage of ovulation induction treatment, preferably a difference between an serum inhibin B concentration on day 6 of menstruation and an inhibin B concentration in venous blood on day 2 of menstruation of the female subject in a cycle of ovulation induction treatment by GnRH antagonist regimen.

29. The method according to any one of claims 23 to 28, wherein

in the data collection step, data of the age, the basal anti-Müllerian hormone (AMH) level, the dynamic change in inhibin B level ($\Delta$INHB), and the basal antral follicle count ( AFC) of the subject is acquired; and
in the calculation step for obtaining the dosage of the exogenous FSH drug, a first calculation is performed on the data acquired by the data collection step, so as to calculate the predicted number of retrieved oocytes (predicted NROs) of the subject in the ovulation induction cycle; a second calculation is performed on the data acquired by the data collection step, so as to calculate a ratio of the predicted number of retrieved oocytes (predicted NROs) of the subject in the ovulation induction cycle to an adjusted dosage of the exogenous FSH drug, i.e., predicted ovarian sensitivity; and the adjusted dosage of the exogenous FSH drug to be administered to the subject is calculated based on the predicted number of retrieved oocytes (predicted NROs) obtained by the first calculation and the ratio obtained by the second calculation.

30. The method according to claim 29, wherein
in the calculation step for obtaining the dosage of the exogenous FSH drug, formula I for calculating the predicted number of retrieved oocytes (predicted NROs) of the subject is pre-stored, wherein the formula I is obtained by fitting based on data of the age, the basal anti-Miillerian hormone (AMH) level, the dynamic change in inhibin B level ($\Delta$INHB), the basal antral follicle count (AFC) and the actual number of retrieved oocytes of a patient who has received ovulation induction treatment by standard GnRH antagonist regimen in an existing database.

31. The method according to claim 30, wherein,

in the calculation step for obtaining the dosage of the exogenous FSH drug, the formula I is a calculation formula I obtained by using negative binomial distribution of the outcome variable to fit the data of the age, the basal anti-Müllerian hormone (AMH) level, the basal follicle-stimulating hormone (FSH), the basal antral follicle count (AFC) and the actual number of retrieved oocytes (outcome variable) of the patient who has received ovulation induction treatment by standard GnRH antagonist regimen in the existing database,; and
the formula I can be used to calculate the predicted number of retrieved oocytes (predicted NROs) of the subject by using the data of the age, the basal anti-Miillerian hormone (AMH) level, the basal follicle-stimulating hormone (FSH) level, and the basal antral follicle count (AFC) of the subject acquired by the data collection step.

32. The method according to claim 31, wherein the formula I is:

$$\text{the predicted NROs} = EXP(a + b*age* + c*basal\ FSH + d*LN[basal\ AMH] + f*LN[basal\ AFC]);$$

wherein a is any value selected from 1.5576128 to 2.6037078, preferably 2.0806603;
b is any value selected from -0.019097 to 0.0044064, preferably -0.007345;
c is any value selected from -0.045234 to -0.004054, preferably -0.024644;
d is any value selected from 0.348168 to 0.4948875, preferably 0.4215277; and
f is any value selected from 0.0415663 to 0.2566199, preferably 0.1490931.

33. The method according to claim 32, wherein

in the calculation step for obtaining the dosage of the exogenous FSH drug, formula II for calculating the predicted ovarian sensitivity of the subject, i.e. the ratio of the predicted number of retrieved oocytes (predicted NROs) to the initial dosage of the exogenous FSH drug is pre-stored, wherein the formula II is obtained by fitting based on the data of the age, the basal anti-Müllerian hormone (AMH) level, the basal follicle-stimulating hormone (FSH) , and the basal antral follicle count (AFC) of the patient who has received ovulation induction treatment by standard GnRH antagonist regimen in the existing database, and a ratio of the predicted number of retrieved oocytes (predicted NROs) calculated by formula I to an average daily dosage of the exogenous FSH drug used by the patient;

wherein the average daily dosage of the exogenous FSH drug used by the patient refers to a ratio of a total dosage of the exogenous FSH drug used by the patient to the number of days of using the exogenous FSH drug during the past ovulation induction treatment by standard GnRH antagonist regimen.

34. The method according to claim 33, wherein

in the calculation step for obtaining the dosage of the exogenous FSH drug, the formula II can be used to calculate the predicted ovarian sensitivity of the subject, i.e., the ratio of the predicted numbers of retrieved oocytes (predicted NROs) to the initial dosage of the exogenous FSH drug to be administrated to the subject, by using the data of the age, the basal anti-Müllerian hormone (AMH) level, the basal follicle-stimulating hormone (FSH) level, and the basal antral follicle count (AFC) of the subject acquired by the data collection step.

35. The method according to claim 33 or 34, wherein the formula II is:

the predicted ovarian sensitivity of the subject, i.e., the predicted NROs calculated by formula I/ the initial dosage of the exogenous FSH drug= EXP(g+h*age+i*basal FSH+j*LN[basal AMH]+k*basal AFC)
wherein g is any value selected from -3.167587 to -2.751518, preferably -2.959552;
h is any value selected from -0.025951 to -0.016355, preferably -0.021153;
i is any value selected from -0.048143 to -0.025727, preferably -0.036935;
j is any value selected from 0.5174476 to 0.6075545, preferably 0.5625011; and
k is any value selected from 0.0241595 to 0.0365579, preferably 0.0303587.

36. The method according to claim 35, wherein based on the predicted numbers of retrieved oocytes (predicted NROs) obtained by the first calculation and the ratio obtained by the second calculation, the initial dosage of the exogenous FSH drug to be administered to the subject is calculated by using formula III, which is:
the initial dosage of the exogenous FSH drug = Round (the predicted NROs calculated by formula I/the predicted ovarian sensitivity calculated by formula II, 0).

37. The method according to any one of claims 23 to 28, wherein

in the data collection step, data of the age, the basal anti-Müllerian hormone (AMH) level, the basal antral follicle count (AFC) and the dynamic change in inhibin B level ($\Delta$INHB) of the subject is acquired; and
in the calculation step for obtaining the dosage of the exogenous FSH drug, a first calculation is performed on the data acquired by the data collection module, so as to calculate the predicted number of retrieved oocytes (predicted NROs) of the subject in the ovulation induction cycle; a second calculation is performed on the data acquired by the data collection step, so as to calculate a ratio of the predicted number of retrieved oocytes (predicted NROs) of the subject in the ovulation induction cycle to an adjusted dosage of the exogenous FSH drug, i.e., predicted ovarian sensitivity; and the adjusted dosage of the exogenous FSH drug to be administered to the subject is calculated based on the predicted number of retrieved oocytes (predicted NROs) obtained by the first calculation and the ratio obtained by the second calculation.

38. The method according to claim 37, wherein
in the calculation step for obtaining the dosage of the exogenous FSH drug, formula IV for calculating the predicted number of retrieved oocytes (predicted NROs) of the subject is pre-stored, wherein the formula IV is obtained by fitting based on the data of the age, the basal anti-Miillerian hormone (AMH) level, the basal antral follicle count (AFC) and the dynamic change in inhibin B level ($\Delta$INHB), and the actual number of retrieved oocytes of a patient who has received ovulation induction treatment by standard GnRH antagonist regimen in the existing database.

39. The method according to claim 38, wherein

in the calculation step for obtaining the dosage of the exogenous FSH drug, the formula IV is a calculation formula IV obtained by using negative binomial distribution of the outcome variable to fit the data of the age, the basal anti-Miillerian hormone (AMH) level, the basal antral follicle count (AFC) and the dynamic change in inhibin B level ($\Delta$INHB), and the actual number of retrieved oocytes (outcome variable) of the patient who has received ovulation induction treatment by standard GnRH antagonist regimen in the existing database;

the formula IV can be used to calculate the predicted number of retrieved oocytes (predicted NROs) of the subject by using the data of the age, the basal anti-Miillerian hormone (AMH) level, the basal antral follicle count (AFC) and the dynamic change in inhibin B level (ΔINHB) of the subject acquired by the data collection step.

40. The method according to claim 39, wherein the formula IV is:

the predicted NROs=EXP(w+m*age*+n*LN[basal AMH]+o*LN[ΔINHB]+p*LN[basal AFC]);

wherein w is any value selected from -0.447201 to 0.9161863, preferably 0.2344927;
m is any value selected from -0.017165 to 0.0039328, preferably -0.006616;
n is any value selected from 0.1318094 to 0.3113979, preferably 0.2216036;
o is any value selected from 0.1901643 to 0.3850919, preferably 0.2876281; and
p is any value selected from 0.0541966 to 0.2338079, preferably 0.1440023.

41. The method according to claim 40, wherein

in the calculation step for obtaining the dosage of the exogenous FSH drug, formula V for calculating the predicted ovarian sensitivity of the subject, i.e. the ratio of the predicted number of retrieved oocytes (predicted NROs) to the adjusted dosage of the exogenous FSH drug, is pre-stored; wherein the formula V is obtained by fitting based on the data of the age, the basal anti-Miillerian hormone (AMH) level, the basal antral follicle count (AFC), and the dynamic change in inhibin B level (ΔINHB) of the patient who has received ovulation induction treatment by standard GnRH antagonist regimen in the existing database, and a ratio of predicted number of retrieved oocytes (predicted NROs) calculated by formula IV to an average daily dosage of the exogenous FSH drug used by the patient,
wherein the average daily dosage of the exogenous FSH drug used by the patient refers to a ratio of a total dosage of the exogenous FSH drug used by the patient to the number of days of using the exogenous FSH drug during the past ovulation induction treatment by standard GnRH antagonist regimen.

42. The method according to claim 41, wherein
in the calculation step for obtaining the dosage of the exogenous FSH drug, the formula V can be used to calculate the predicted ovarian sensitivity of the subject, i.e., the ratio of the predicted number of retrieved oocytes (predicted NROs) to the adjusted dosage of the exogenous FSH drug to be administrated to the subject, by using the data of the age, the basal anti-Müllerian hormone (AMH) level, the basal antral follicle count (AFC) and the dynamic change in inhibin B level (ΔINHB) of the subject acquired by the data collection step.

43. The method according to claim 41 or 42, wherein the formula V is:

the predicted ovarian sensitivity of the subject, i.e., the predicted NROs calculated by formula IV/ the adjusted dosage of the exogenous FSH drug = EXP(q+r*age+s*LN[basal AMH]+t*basal AFC+u*LN[ΔINHB])
wherein q is any value selected from -5.63461 to -5.108612, preferably -5.371611;
r is any value selected from -0.0264 to -0.015183, preferably -0.020792;
s is any value selected from 0.2696292 to 0.3684551, preferably 0.3190421;
t is any value selected from 0.0273504 to 0.0399372, preferably 0.0336438; and
u is any value selected from 0.3327566 to 0.3940448, preferably 0.3634007.

44. The method according to claim 43, wherein based on the predicted number of retrieved oocytes (predicted NROs) obtained by the first calculation and the ratio obtained by the second calculation, the adjusted dosage of the exogenous FSH drug to be administered to the subject is calculated by using Formula VI which is:
the adjusted dosage of the exogenous FSH drug = Round (the predicted NROs calculated by formula VI/the predicted ovarian sensitivity calculated by formula V, 0).

Distribution before transformation
of the outcome variable:

Figure.1A

Distribution after transformation
of the outcome variable:

Figure.1B

Relationship between AMH and the outcome variable

Figure.1C

Figure.2A

Figure.2B

|  | Main contribution(%) | Total contribution(%) |
|---|---|---|
| AMH | 83.5 | 86.0 |
| AFC | 7.5 | 9.9 |
| Basal FSH | 1.5 | 2.4 |
| Age | 1.5 | 2.5 |

Figure.2C

Figure.2D

Figure.2E

Figure.2F

Figure.2G

Figure.3A

Figure.3B

|  | Main contribution(%) | Total contribution(%) |
|---|---|---|
| Δ Inhibin B | 48.9 | 50.8 |
| AMH | 30.0 | 31.8 |
| AFC | 12.7 | 14.5 |
| Age | 2.0 | 3.2 |

Figure.3C

Figure.3D

Figure.3E

Figure.3F

Figure.3G

## INTERNATIONAL SEARCH REPORT

| International application No. |
| --- |
| **PCT/CN2022/111753** |

**A.　CLASSIFICATION OF SUBJECT MATTER**

　　G16H 50/20(2018.01)i

According to International Patent Classification (IPC) or to both national classification and IPC

**B.　FIELDS SEARCHED**

Minimum documentation searched (classification system followed by classification symbols)

　　G16H

Documentation searched other than minimum documentation to the extent that such documents are included in the fields searched

Electronic data base consulted during the international search (name of data base and, where practicable, search terms used)

　　EPODOC, WPI, CNPAT, CNKI, IEEE, GOOGLE: 预测, 卵巢, 刺激, 周期, 外源性, 卵泡刺激素, 药物剂量, 采集, prediction, ovary, stimulation, cycle, exogenous, follicle stimulating hormone, drug dose, collect

**C.　DOCUMENTS CONSIDERED TO BE RELEVANT**

| Category* | Citation of document, with indication, where appropriate, of the relevant passages | Relevant to claim No. |
| --- | --- | --- |
| A | CN 110491505 A (PEKING UNIVERSITY THIRD HOSPITAL (PEKING UNIVERSITY THIRD CLINICAL MEDICAL COLLEGE)) 22 November 2019 (2019-11-22)<br>　　description, paragraphs 0111-0130, and figures 1-4 | 1-44 |
| A | CN 110570952 A (PEKING UNIVERSITY THIRD HOSPITAL) 13 December 2019 (2019-12-13)<br>　　entire document | 1-44 |
| A | CN 111613289 A (THE FIRST AFFILIATED HOSPITAL OF COLLEGE OF MEDICINE, ZHEJIANG UNIVERSITY et al.) 01 September 2020 (2020-09-01)<br>　　entire document | 1-44 |
| A | US 2021043271 A1 (WOMEN & INFANTS HOSPITAL OF RHODE ISLAND) 11 February 2021 (2021-02-11)<br>　　entire document | 1-44 |

☐ Further documents are listed in the continuation of Box C.　　☑ See patent family annex.

| | |
| --- | --- |
| *　　Special categories of cited documents:<br>"A"　document defining the general state of the art which is not considered to be of particular relevance<br>"E"　earlier application or patent but published on or after the international filing date<br>"L"　document which may throw doubts on priority claim(s) or which is cited to establish the publication date of another citation or other special reason (as specified)<br>"O"　document referring to an oral disclosure, use, exhibition or other means<br>"P"　document published prior to the international filing date but later than the priority date claimed | "T"　later document published after the international filing date or priority date and not in conflict with the application but cited to understand the principle or theory underlying the invention<br>"X"　document of particular relevance; the claimed invention cannot be considered novel or cannot be considered to involve an inventive step when the document is taken alone<br>"Y"　document of particular relevance; the claimed invention cannot be considered to involve an inventive step when the document is combined with one or more other such documents, such combination being obvious to a person skilled in the art<br>"&"　document member of the same patent family |

| Date of the actual completion of the international search | Date of mailing of the international search report |
| --- | --- |
| **18 November 2022** | **28 November 2022** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT
### Information on patent family members

International application No.

**PCT/CN2022/111753**

| Patent document cited in search report | | | Publication date (day/month/year) | Patent family member(s) | | | Publication date (day/month/year) |
|---|---|---|---|---|---|---|---|
| CN | 110491505 | A | 22 November 2019 | WO | 2021031605 | A1 | 25 February 2021 |
| CN | 110570952 | A | 13 December 2019 | WO | 2019233308 | A1 | 12 December 2019 |
| CN | 111613289 | A | 01 September 2020 | None | | | |
| US | 2021043271 | A1 | 11 February 2021 | WO | 2019177892 | A1 | 19 September 2019 |

Form PCT/ISA/210 (patent family annex) (January 2015)